# EUROPÄISCHE PATENTSCHRIFT

(11) **EP 0 625 976 B1**
(45) Veröffentlichungstag und Bekanntmachung des Hinweises auf die Patenterteilung: **13.11.1996**
(21) Anmeldenummer: 93917363.9
(22) Anmeldetag: 30.01.1993
(51) Int. Cl.: C07D 309/06, C07D 213/64, C07D 263/58, C07D 277/68, C07C 251/54, C07D 335/02, A01N 43/78, A01N 43/40, A01N 43/60, C07D 239/20, C07D 409/12

(54) **MISCHUNGEN AUS OPTISCH AKTIVEN CYCLOHEXENONOXIMETHERN, VERFAHREN UND ZWISCHENPRODUKTE ZU IHRER HERSTELLUNG UND IHRE VERWENDUNG ALS HERBIZIDE**
MIXTURES OF OPTICALLY ACTIVE CYCLOHEXENONE OXIME ETHERS, PROCESS AND INTERMEDIATE PRODUCTS FOR THEIR PRODUCTION AND THEIR USE AS HERBICIDES
MELANGES D'ETHERS DE CYCLOHEXENONE-OXIMES OPTIQUEMENT ACTIFS, PROCEDE ET PRODUITS INTERMEDIAIRES NECESSAIRES POUR LEUR FABRICATION ET UTILISATION COMME HERBICIDES

(30) Priorität: 13.02.1992 DE 4204204
(43) Veröffentlichungstag der Anmeldung: 30.11.1994
(73) Patentinhaber: BASF Aktiengesellschaft, 67063 Ludwigshafen (DE)
(72) Erfinder: MISSLITZ, Ulf, D-6730 Neustadt (DE); MEYER, Norbert, D-6802 Ladenburg (DE); KAST, Juergen, D-6737 Boehl-Iggelheim (DE); LADNER, Wolfgang, D-6701 Fussgoenheim (DE); WALTNER, Helmut, D-6719 Obrigheim (DE); WESTPHALEN, Karl-Otto, D-6720 Speyer (DE); KARDORFF, Uwe, D-6800 Mannheim 1 (DE); GERBER, Matthias, D-6704 Mutterstadt (DE)
(86) Internationale Anmeldenummer: EP9300210
(87) Internationale Veröffentlichungsnummer: WO9316061

(56) Entgegenhaltungen:
- EP-A- 0 089 115
- EP-A- 0 177 913
- EP-A- 0 205 821
- EP-A- 0 456 112
- CHEMICAL ABSTRACTS, vol. 102, no. 19, 13. Mai 1985, Columbus, Ohio, US; abstract no. 166459f, Seite 591 ;
- CHEMICAL ABSTRACTS, vol. 111, no. 17, 23. Oktober 1989, Columbus, Ohio, US; abstract no. 153345b, Seite 674 ;

## Beschreibung

Die vorliegende Erfindung betrifft neue Mischungen aus optisch aktiven Cyclohexenonoximethern mit R- und S-Konfiguration im Oximetherteil der allgemeinen Formel I 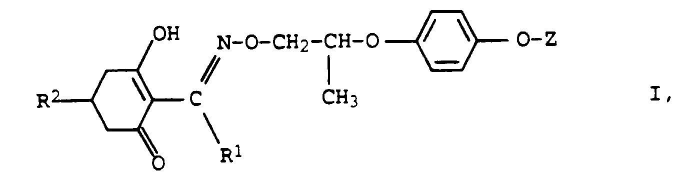 in der die Variablen folgende Bedeutung haben:
- R¹: eine C₁-C₆-Alkylgruppe;
- Z: eine der folgenden Gruppen: 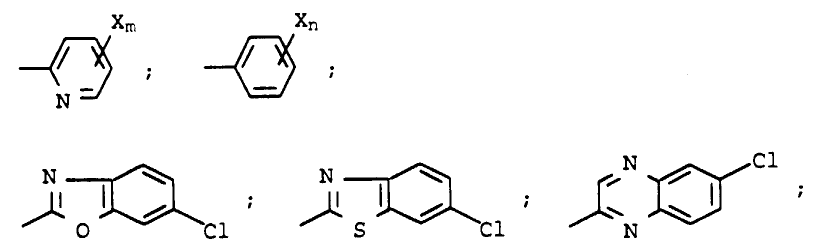
- X: Halogen, C₁-C₄-Halogenalkyl;
- m: 0 bis 3 oder 1 bis 4 für den Fall, daß alle X Halogen bedeuten;
- n: 0 bis 3 oder 1 bis 5 für den Fall, daß alle X Halogen bedeuten;
- R²: eine C₁-C₄-Alkoxy-C₁-C₆-alkyl- oder C₁-C₄-Alkylthio-C₁-C₆-alkylgruppe;
eine C₃-C₇-cycloalkylgruppe oder eine C₅-C₇-cycldalkenylgruppe, wobei diese Gruppen gewünschtenfalls ein bis drei Substituenten tragen können, ausgewählt aus einer Gruppe bestehend aus C₁-C₄-Alkyl, C₁-C₄-Alkoxy, C₁-C₄-Alkylthio, C₁-C₄-Halogenalkyl, Hydroxyl und Halogen;
ein 5-gliedriger gesättigter Heterocyclus, der ein oder zwei Sauerstoff- und/oder Schwefelatome als Heteroatome enthält und der gewünschtenfalls noch ein bis drei Substituenten tragen kann, ausgewählt aus einer Gruppe bestehend aus C₁-C₄-Alkyl, C₁-C₄-Alkoxy, C₁-C₄-Alkylthio und C₁-C₄-Halogenalkyl;
ein 6- oder 7-gliedriger gesättigter oder ein- oder zweifach ungesättigter Heterocyclus, der ein- oder zwei Sauerstoff- oder Schwefelatome oder ein Sauerstoff- und ein Schwefelatom als Heteroatome enthält,
   wobei der Heterocyclus gewünschtenfalls noch ein bis drei Substituenten tragen kann, ausgewählt aus einer Gruppe bestehend aus Hydroxyl, Halogen, C₁-C₄-Alkyl, C₁-C₄-Alkoxy, C₁-C₄-Alkylthio und C₁-C₄-Halogenalkyl;
ein 5-gliedriger Heteroaromat, enthaltend ein bis drei Heteroatome, ausgewählt aus einer Gruppe bestehend aus ein oder zwei Stickstoffatomen und ein Sauerstoff- oder Schwefelatom, wobei der Heteroaromat gewünschtenfalls noch ein bis drei Substituenten tragen kann, ausgewählt aus einer Gruppe bestehend aus Halogen, Cyano, C₁-C₄-Alkyl, C₁-C₄-Alkoxy, C₁-C₄-Alkylthio, C₁-C₄-Halogenalkyl, C₂-C₆-Alkenyl, C₂-C₆-Alkenyloxy und C₁-C₄-Alkoxy-C₁-C₄-alkyl;
eine Phenyl- oder Pyridylgruppe, wobei diese Aromaten gewünschtenfalls noch ein bis drei Substituenten tragen können ausgewählt aus einer Gruppe bestehend aus Halogen, Nitro, Cyano, C₁-C₄-Alkyl, C₁-C₄-Alkoxy, C₁-C₄-Alkylthio, C₁-C₄-Halogenalkyl, C₃-C₆-Alkenyloxy, C₃-C₆-Alkinyloxy und einer Aminogruppe -NR^{a}R^{b}, worin
   R^{a} Wasserstoff, C₁-C₄-Alkyl, C₃-C₆-Alkenyl oder C₃-C₆-Alkinyl und
   R^{b} Wasserstoff, C₁-C₄-Alkyl, C₃-C₆-Alkenyl, C₃-C₆-Alkinyl, C₁-C₆-Acyl oder Benzoyl, das gewünschtenfalls seinerseits noch ein bis drei Reste tragen kann, ausgewählt aus einer Gruppe bestehend aus Nitro, Cyano, Halogen, C₁-C₄-Alkyl, C₁-C₄-Alkoxy, C₁-C₄-Alkylthio und C₁-C₄-Halogenalkyl;
sowie der landwirtschaftlich brauchbaren Salze und Ester von C₁-C₁₀-Carbonsäuren und anorganischen Säuren der Verbindungen I.

Außerdem betrifft die Erfindung ein Verfahren zur Herstellung dieser Verbindungen, ihre Verwendung als Herbizide sowie herbizide Mittel, welche diese Mischungen enthalten.

Des weiteren betrifft die Erfindung neue Mischungen aus optisch aktiven Hydroxylaminen mit R- und S-Konfiguration der Formel III 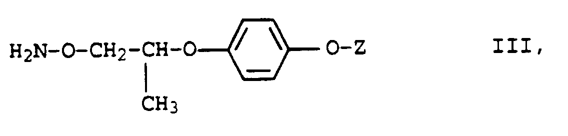 in der Z eine der folgenden Gruppen bedeutet: 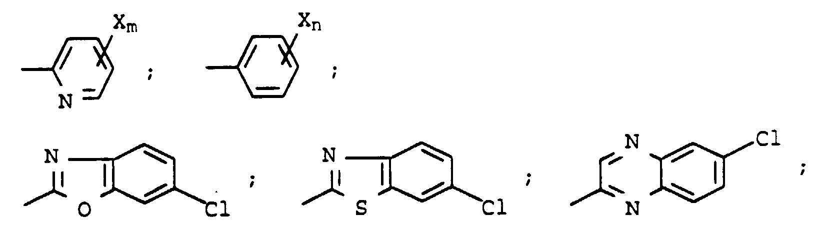 wobei
- X: für Halogen oder C₁-C₄-Halogenalkyl,
- m: für 0 bis 3 oder 1 bis 4 für den Fall, daß alle X Halogen bedeuten, und
- n: für 0 bis 3 oder 1 bis 5 für den Fall, daß alle X Halogen bedeuten,
stehen.

Aus der Literatur sind bereits herbzid wirksame Cyclohexandione der Formel I' 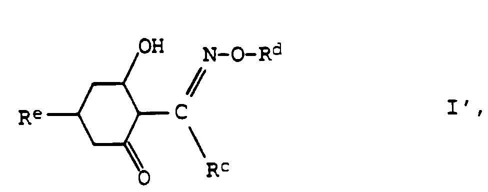 bekannt, wobei R^{c}, R^{d} und R^{e} u.a. für die folgenden Bedeutungen stehen:
- US 4,440,566 (R^{c} = Ethyl, Propyl; R^{d} = Benzyl; R^{e} = 2-Ethylthiopropyl);
- EP-A 238 021 und EP-A 125 094 (R^{c} = Ethyl, Propyl; R^{d} = Benzyl, But-2-enyl; R^{e} = substituierter 5-gliedriger Heteroarylrest);
- EP-A 80 301 (R^{c} = Ethyl, Propyl; R^{d} = Benzyl, But-2-enyl; R^{e} = substituiertes Phenyl);
- DE-A 38 38 309 (R^{c} = Ethyl, Propyl; R^{d} = substituierter 4-Phenylbutylen- oder 4-Phenylbutenylenrest; R^{e} = substituierter 5- bis 7-glieriger Heterocyclus);
- EP-A 456 112 (R^{c} = Ethyl, Propyl; R^{d} = substituierter 3-Phenoxypropylen- oder 2-Phenoxyethylenrest; R^{e} = substituierter 5-bis 7-gliedriger Heterocyclus).

Die herbiziden Eigenschaften dieser Verbindungen, insbesondere bezüglich ihrer Selektivität gegen Ungräser in grasartigen Kulturpflanzen, können jedoch nur bedingt befriedigen.

Der Erfindung lagen daher neue Mischungen aus Cyclohexenon-oximethern mit verbesserter Selektivität gegen Ungräser in grasartigen Kulturen wie Reis und Mais zugrunde.

Demgemäß wurden die eingangs definierten Mischungen aus optisch aktiven Cyclohexenonoximethern I gefunden. Außerdem wurden herbizide Mittel gefunden, die diese Mischungen enthalten.

Die Mischungen aus optisch aktiven Cyclohexenonoximethern I sind auf verschiedene Weise erhältlich, und zwar bevorzugt in an sich bekannter Weise aus schon bekannten Cyclohexenonen der Formel II, (DE-A 38 38 309, EP-A 243 313, EP-A 456 112) und den entsprechenden Mischungen aus optisch aktiven Hydroxylaminen mit R- und S-Konfiguration der Formel III (vgl. EP-A 169 521):

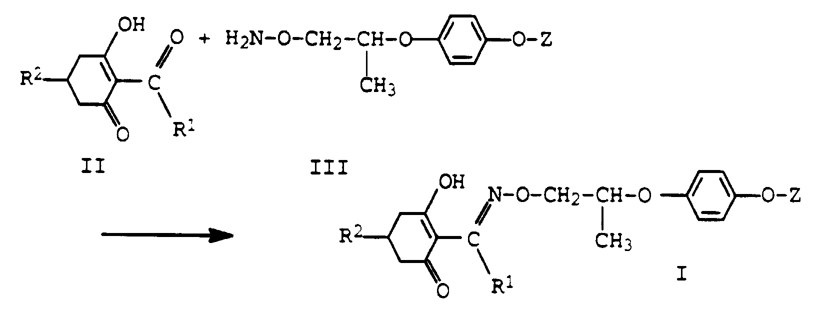

Vorzugsweise verwendet man ein geeignetes Salz der Hydroxylamine III, insbesondere deren Hydrochlorid, und führt die Reaktion in heterogener Phase in einem inerten Lösungsmittel durch, beispielsweise in Dimethylsulfoxid, in einem Alkohol wie Methanol, Ethanol und Isopropanol, in einem aromatischen Kohlenwasserstoff wie Benzol und Toluol, in einem chlorierten Kohlenwasserstoff wie Chloroform und 1,2-Dichlorethan, in einem aliphatischen Kohlenwasserstoff wie Hexan und Cyclohexan, in einem Ester wie Essigsäureethylester oder in einem Ether wie Diethylether, Dioxan und Tetrahydrofuran.

Die Reaktionsführung erfolgt in Gegenwart einer Base, wobei normalerweise eine Basenmenge von etwa 0,5 bis 2 Mol-Äquivalent, bezogen auf die Ammoniumverbindung, ausreichend ist.

Als Basen kommen z.B. Carbonate, Hydrogencarbonate, Acetate, Alkoholate oder Oxide von Alkali- oder Erdalkalimetallen, insbesondere Natriumhydroxid, Kaliumhydroxid, Magnesiumoxid oder Calciumoxid in Betracht. Des weiteren sind organische Basen wie Pyridin und tert. Amine wie Triethylamin geeignet.

Vorzugsweise führt man die Umsetzung in Methanol mit Natriumhydrogencarbonat als Base durch.

Eine Variante des Verfahrens besteht darin, die Umsetzung ohne Base mit den freien Hydroxylaminbasen III, z.B. in Form einer wäßrigen Lösung, vorzunehmen; je nach verwendetem Lösungsmittel für die Verbindung II erhält man ein ein- oder zweiphasiges Reaktionsgemisch.

Geeignete Lösungsmittel für diese Variante sind beispielsweise Alkohole wie Methanol, Ethanol, Isopropanol und Cyclohexanol, aliphatische und aromatische, gegebenenfalls chlorierte Kohlenwasserstoffe wie Hexan, Cyclohexan, Methylenchlorid, Toluol und 1,2-Dichlorethan, Ester wie Essigsäureethylester, Nitrile wie Acetonitril und cyclische Ether wie Dioxan und Tetrahydrofuran.

Zweckmäßigerweise setzt man das Cyclohexenon II und die Mischung aus optisch aktiven Hydroxylaminen III bzw. deren Salze in etwa stöchiometrischem Verhältnis ein, jedoch kann in manchen Fällen auch ein Überschuß der einen oder anderen Komponente, bis ca. 10 mol-%, vorteilhaft sein.

Die Reaktionstemperatur liegt im allgemeinen zwischen 0°C und dem Siedepunkt des Reaktionsgemisches, vorzugsweise zwischen 20 und 80°C.

Die Reaktion ist nach wenigen Stunden beendet. Das Produkt kann auf übliche Weise, z.B. durch Einengen der Mischung, Verteilung des Rückstandes in Methylenchlorid/Wasser und Abdestillieren des Lösungsmittels unter vermindertem Druck, isoliert werden.

Besondere Bedingungen bezüglich des Drucks sind nicht zu beachten; im allgemeinen arbeitet man daher bei Normaldruck oder unter dem Eigendruck des jeweiligen Verdünnungsmittels.

Aufgrund ihres sauren Charakters können die optisch aktiven Cyclohexenonoximether I Salze von Alkali- oder Erdalkalimetallverbindungen sowie Enolester bilden.

Alkalimetallsalze der Verbindungen I können durch Behandelnder 3-Hydroxycyclohexenon-Verbindungen mit Natrium- oder Kaliumhydroxid bzw. -alkoholat in wäßriger Lösung oder in einem organischen Lösungsmittel wie Methanol, Ethanol, Aceton und Toluol erhalten werden.

Andere Metallsalze wie Mangan-, Kupfer-, Zink-, Eisen-, Calcium-, Magnesium- und Bariumsalze können aus den Natriumsalzen in üblicher Weise hergestellt werden, ebenso Ammonium- und Phosphoniumsalze mittels Ammoniak, Phosphonium-, Sulfonium- oder Sulfoxoniumhydroxiden.

Die Ester der Verbindungen I sind ebenfalls in üblicher Weise erhältlich (vgl. z.B. Organikum, VEB Deutscher Verlag der Wissenschaften, 17. Auflage, Berlin 1988, S. 405-408).

Die neuen Mischungen aus optisch aktiven Hydroxylaminen III lassen sich über eine Reihe bekannter Verfahrensschritte ausgehend von bekannten Vorprodukten herstellen: 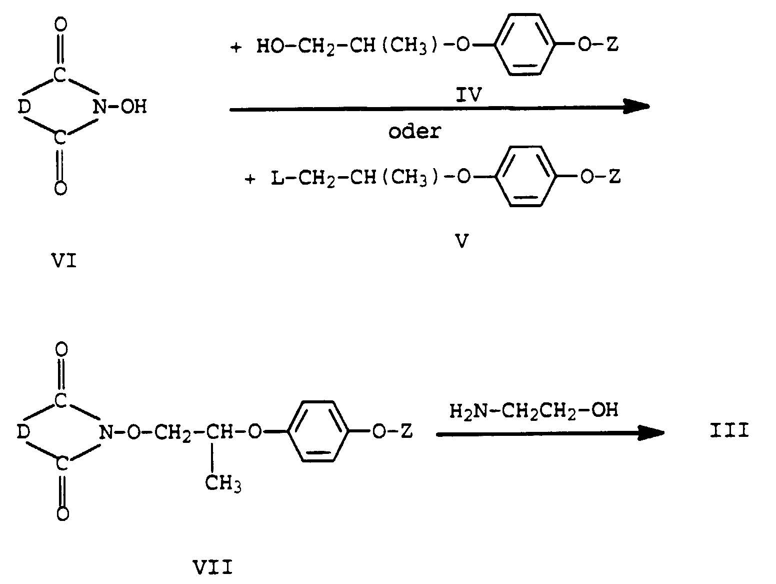 L = eine Abgangsgruppe, z.B. Halogen wie Chlor, Brom und Iod oder CH₃SO₂-O-.

Vorzugsweise koppelt man das optisch aktive Alkylierungsmittel V {Z. Naturforsch. 37 B, 912 (1982); DE-A 26 11 695}, gewünschtenfalls aber auch das optisch aktive Carbinol IV {Z. Naturforsch. 37 B, 912 (1982); DE-A 26 11 695; US 4,491,468; EP-A 003 877; DE-A 25 43 179; DE-A 26 49 706; DE-A 24 15 867) nach der Mitsunobu-Variante (Synthesis 1, (1981); J. Med. Chem. 33, 187 (1990)}, mit einem cyclischen Hydroxyimid VI und spaltet das hierbei erhaltene geschützte Hydroxylaminderivat VII zum freien Hydroxylamin III, z.B. mit 2-Aminoethanol.

In den cyclischen Hydroxyimiden VI steht D z.B. für C₂-C₃-Alkylen, C₂-Alkenylen oder einen 5- oder 6- gliedrigen Ring mit gegebenenfalls einem Stickstoffatom, der gesättigt, teilweise ungesättigt oder aromatisch sein kann, z.B. für Phenylen, Pyridinylen, Cyclopentylen, Cyclohexylen oder Cyclohexenylen.

Beispielsweise kommen folgende Substanzen in Betracht:

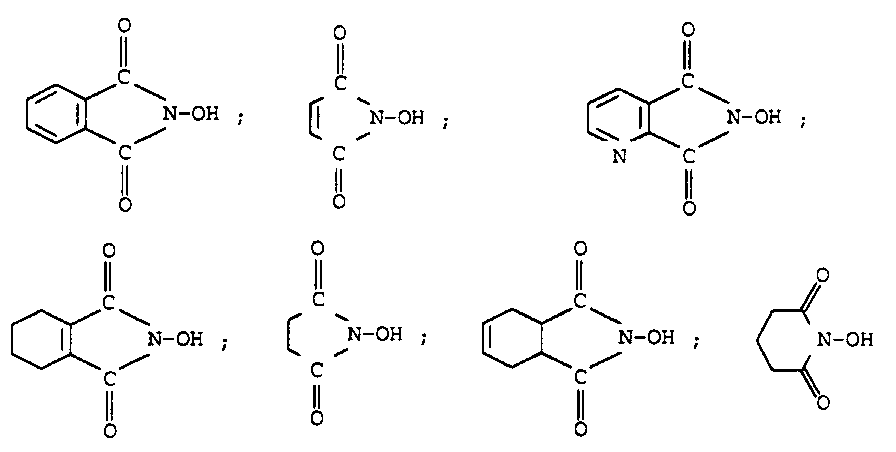

Die Umsetzung der optisch aktiven Alkylierungsmittel V mit den Hydroxyimiden VI wird zweckmäßigerweise in Gegenwart einer Base durchgeführt. Geeignet sind prinzipiell alle Basen, die in der Lage sind, die Hydroxyimide VI zu deprotonieren, ohne das Imidsystem anzugreifen. Dies sind insbesondere die sogenannten nichtnucleophilen Basen.

Beispielsweise genannt seien Mineralbasen wie Alkalimetall- und Erdalkalimetallcarbonate, Alkalimetall- und Erdalkalimetallhydrogencarbonate, organische Basen wie aliphatische, cycloaliphatische und aromatische tertiäre Amine. Es können aber auch Gemische dieser Basen verwendet werden.

Als Einzelverbindungen seien folgende Basen beispielhaft aufgeführt: Natriumcarbonat, Kaliumcarbonat, Magnesiumcarbonat, Calciumcarbonat, Bariumcarbonat, die Hydrogencarbonate dieser Metalle, Trimethylamin, Triethylamin, Tributylamin, Ethyldiisopropylamin, N,N-Dimethylanilin, 4-(N,N-Dimethylamino)pyridin, Diazabicyclooctan, Diazabicycloundecan, N-Methylpiperidin, 1,4-Dimethylpiperazin, Pyridin, Chinolin, Bipyridin, Phenanthrolin. Bevorzugt sind die preiswerten Basen Natrium- und Kaliumcarbonat.

Die Base wird im allgemeinen in äquivalenten Mengen bis zu einem Überschuß von 5 Äquivalenten, bezogen auf das Hydroxyimid, zugegeben. Ein höherer Überschuß ist möglich, bringt aber in der Regel keine zusätzlichen Vorteile. Die Verwendung einer geringen Basenmenge ist ebenfalls möglich. Bevorzugt wird jedoch eine Basenmenge von 1 bis 3, insbesondere von 1 bis 2 Äquivalenten, bezogen auf das Hydroxyimid VI eingesetzt.

Die Verwendung von nucleophilen Basen, z.B. Alkalimetall- und Erdalkalimetallhydroxiden, insbesondere Natrium- und Kaliumhydroxid, ist ebenfalls möglich. In diesem Falle ist es vorteilhaft, die Base in äquivalenten Mengen bezüglich des Hydroxyimids VI einzusetzen, um einem nucleophilen Angriff der Hydroxylionen auf die Carbonylfunktion der Imidgruppierung vorzubeugen.

Zweckmäßigerweise setzt man die optisch aktiven Alkylierungsmittel V mit den Hydroxyimiden VI in einem Lösungsmittel um, das sich unter den Reaktionsbedingungen inert verhält. Vorteilhafte Lösungsmittel sind z.B. polare, aprotische Lösungsmittel wie Dimethylformamid, N-Methylpyrrolidon, Dimethylsulfoxid, Sulfolan und cyclische Harnstoffe. Die Lösungsmittelmenge ist im allgemeinen nicht kritisch.

Die Umsetzung der optisch aktiven Alkylierungsmittel V mit den Hydroxyimiden VI kann auch unter Anwendung der Phasentransfer-Katalyse ausgeführt werden. In diesem Falle werden mit Wasser zwei Phasen bildende Lösungsmittel, bevorzugt Chlorkohlenwasserstoffe, eingesetzt.
Als Phasentransferkatalysatoren eignen sich die üblicherweise zu solchen Zwecken verwendeten quartären Ammonium- und Phosphoniumsalze, Polyethylenglykole, Polyethylenglykolether und Kronenether, wie sie z.B. in Dehmlow et al., Phase Transfer Catalysis, S. 37-45 und S. 86-93, Verlag Chemie, Weinheim 1980, beschrieben sind.

Die Phasentransferkatalysatoren werden zweckmäßigerweise in Mengen von 1 bis 10 Vol%, bevorzugt in Mengen von 3 bis 5 Vol%, bezogen auf das Volumen der Reaktionsmischung, eingesetzt.

Die Umsetzung der optisch aktiven Alkylierungsmittel V mit den Hydroxyimiden VI erfolgt im allgemeinen im Temperaturbereich zwischen 0 und 140°C, bevorzugt zwischen 2 und 100°C, insbesondere zwischen 40 und 80°C. Vorteilhaft wird dabei so vorgegangen, daß man das Hydroxyimid VI zusammen mit der Base im Lösungsmittel vorlegt und das Alkylierungsmittel V zu dieser Lösung dosiert. Dabei kann es sich als günstig erweisen, wenn das Hydroxyimid bei einer tieferen Temperatur, beispielsweise bei 0 bis 50°C, zugegeben und die Reaktionsmischung erst nach dieser Zugabe auf die eigentliche Reaktionstemperatur erhitzt wird.

Nach beendeter Reaktion wird die abgekühlte Reaktionsmischung zweckmäßigerweise mit Wasser versetzt, wobei sich die gebildeten Hydroxylaminderivate VII als kristalline Festkörper oder als Öle abscheiden. Die auf diese Weise erhaltenen Hydroxylaminderivate können, falls gewünscht, durch Umkristallisation oder durch Extraktion weiter gereinigt werden.

Die Hydroxylaminderivate VII können zwischengelaget werden oder sogleich in die optisch aktiven Hydroxylamine III mit freier Aminogruppe umgewandelt werden.

Diese Umwandlung kann nach an sich bekannten Verfahren durchgeführt werden, wie sie beispielsweise in DE-A 36 15 973 und den darin zitierten Schriften beschrieben sind. Bevorzugt wird das Verfahren gemaß DE-A 36 15 973 angewandt, nachdem die optisch aktiven Hydroxylamine III mittels Ethanolamin freigesetzt wurden. Die Freisetzung der Hydroxylamine III mit Hilfe anderer Basen wie wäßrigen Mineralbasen, mit Aminen, Hydrazinen, Hydroxylaminen oder mittels wäßriger Säuren ist ebenfalls möglich.

Aus den nach diesen Verfahren erhaltenen Reaktionsgemischen können die optisch aktiven Hydroxylamine III mittels üblicher Aufarbeitungsmethoden isoliert weren, beispielsweise durch Extraktion oder durch Kristallisation. Zur Erhöhung der Kristallisationstendenz dieser Hydroxylamine III kann es oftmals förderlich sein, diese in ihre Salze mit Mineralsäuren oder organischen Säuren überzuführen. Dazu werden im allgemeinen verdünnte Lösungen dieser Säuren mit den Hydroxylaminderivaten umgesetzt, und zwar zweckmäßigerweise in etwa äquivalenten Mengen. Die erhaltenen Hydroxylammoniumsalze können wie die optisch aktiven Hydroxylamine III (mit freier Aminogruppe) direkt zu den optisch aktiven Cyclohexenonoximethern der Formel I weiterverarbeitet werden oder auch, falls gewünscht, gelagert werden.

Die optische Reinheit der Zwischenprodukte III und der Cyclohexenonoximether I hängt von der optischen Reinheit der eingesetzten Carbinole IV bzw. Alkylierungsmittel V ab. Vorzugsweise werden die Carbinole IV bzw. die Alkylierungsmittel V als Mischungen eingesetzt, und zwar mit einem Mindestgehalt an R-Isomeren von 50 mol-%, so daß bei der Herstellung der optisch aktiven Hydroxylamine III und der optisch aktiven Cyclohexenonoximether I jeweils Isomerengemische erhalten werden, deren Anteil an Isomeren mit R-Konfiguration am methylsubstituierten C-Atom (im Oximetherteil) mindestens 50 mol-%, vorzugsweise 90 bis 100 mol-% beträgt.

Je nach Substituenten können die optisch aktiven Cyclohexenonoximether I bei der Herstellung auch als E-/Z-Isomerengemische anfallen, wobei sich die Isomeren durch die Stellung des Oximetherteils relativ zu R¹ unterscheiden. Die E- und Z-Isomeren können gewünschtenfalls nach den hierfür üblichen Methoden, z.B. durch Chromatographie oder durch Kristallisation, getrennt werden.

Die optisch aktiven Cyclohexenonoximether I können in mehreren tautomeren Formen geschrieben werden, die alle von der Erfindung umfaßt werden.

Die in der Definition der Substituenten verwendeten Sammelbegriffe
- Halogen,
- C₁-C₄-Alkyl, C₁-C₄-Alkoxy, C₁-C₄-Alkylthio, C₁-C₄-Halogenalkyl,
- C₂-C₆-Alkenyl, C₂-C₆-Alkenyloxy,
- C₃-C₆-Alkenyl, C₃-C₆-Alkenyloxy, C₃-C₆-Alkinyl, C₃-C₆-Alkinyloxy,
- C₁-C₆-Acyl
stellen Kurzschreibweisen für eine individuelle Aufzählung der einzelnen Gruppenmitglieder dar. Sämtliche Alkyl-, Alkoxy-, Alkylthio-, Halogenalkyl-, Alkenyl-, Alkenyloxy-, Alkinyl- und Alkinyloxyteile können geradkettig oder verzweigt sein. Die Halogenalkylteile können gleiche oder verschiedene Halogenatome tragen.

Im einzelnen bedeuten beispielsweise
- Halogen: Fluor, Chlor, Brom und Jod;
- C₁-C₄-Alkyl: Methyl, Ethyl, n-Propyl, 1-Methylethyl, n-Butyl, 1-Methylpropyl, 2-Methylpropyl und 1,1-Dimethylethyl;
- C₁-C₄-Alkoxy: Methoxy, Ethoxy, n-Propoxy, 1-Methylethoxy, n-Butoxy, 1-Methylpropoxy, 2-Methylpropoxy und 1,1-Dimethylethoxy;
- C₁-C₄-Alkylthio: Methylthio, Ethylthio, n-Propylthio, 1-Methylethylthio, n-Butylthio, 1-Methylpropylthio, 2-Methylpropylthio und 1,1-Dimethylethylthio;
- C₁-C₄-Halogenalkyl: Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl;
- C₂-C₆-Alkenyl: Ethenyl und C₃-C₆-Alkenyl wie 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
- C₂-C₆-Alkenyloxy: Ethenyloxy und C₃-C₆-Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyl-oxy, 3-Methyl-4-pentenyloxy, 4-Methyl-4-pentenyloxy, 1,1-Dimethyl-2-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy und 1-Ethyl-2-methyl-2-propenyloxy.

Im Hinblick auf ihre herbizide Wirksamkeit werden Mischungen aus optisch aktiven Cyclohexenonen der Formel I bevorzugt, in denen die Variablen folgende Bedeutung haben:
- R¹: C₁-C₆-Alkyl wie Methyl, Ethyl, n-Propyl, n-Butyl, n-Pentyl, n-Hexyl, vorzugsweise Ethyl und Propyl;
- Z: eine der folgenden Gruppen: 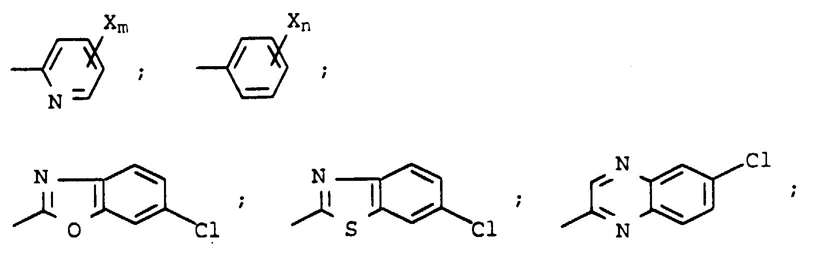 besonders bevorzugt ist 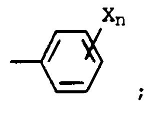
- X: Halogen wie Fluor, Chlor, Brom und Iod, vorzugsweise Fluor, Chlor und Brom;
C₁-C₄-Halogenalkyl, vorzugsweise Difluormethyl, Trifluormethyl, 2,2,2-Trifluorethyl und Pentafluorethyl; besonders bevorzugt sind Halogen und Trifluormethyl;
- m: 0 bis 3 oder 1 bis 4 für den Fall, daß alle X Halogen bedeuten, vorzugsweise 0 bis 3;
- n: 0 bis 3 oder 1 bis 5 für den Fall, daß alle X Halogen bedeuten, vorzugsweise 0 bis 3;
- R²: - eine C₁-C₆-Alkylgruppe wie Methyl, Ethyl, n-Propyl, 1-Methylethyl, n-Butyl, 1-Methylpropyl, 2-Methylpropyl und 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethybutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, wobei die Alkylgruppe durch C₁-C₄-Alkoxy, vorzugsweise Methoxy, Ethoxy, 1-Methylethoxy und 1,1-Dimethylethoxy, oder durch C₁-C₄-Alkylthio, vorzugsweise Methylthio und Ethylthio, substituiert ist, und zwar bevorzugt in 1-, 2-oder 3-Position;
   ganz besonders bevorzugt ist 2-Ethylthiopropyl;
- eine C₃-C₇-Cycloalkylgruppe oder eine C₅-C₇-Cycloalkenylgruppe wie Cyclopropyl, Cyclobutyl, Cyclopentyl, Cyclohexyl, Cycloheptyl, Cyclopentenyl, Cyclohexenyl und Cycloheptenyl, wobei diese Gruppen unsubstituiert sein oder ein bis drei der folgenden Substituenten tragen können:
   C₁-C₄-Alkyl, vorzugsweise Methyl, C₁-C₄-Alkoxy, C₁-C₄-Alkylthio und C₁-C₄-Halogenalkyl;
   ganz besonders bevorzugt ist 1-Methylthio-1-cyclopropyl;
- ein 5-gliedriger gesättigter Heterocyclus wie Tetrahydrofuranyl, Tetrahydrothienyl, Dioxolanyl, Dithiolanyl und Oxathiolanyl, insbesondere Tetrahydrofuranyl, Tetrahydrothienyl und Dioxolanyl, wobei diese Ringe unsubstituiert sein oder ein bis drei Substituenten tragen können, ausgewählt aus einer Gruppe bestehend aus C₁-C₄-Alkyl, C₁-C₄-Alkoxy, C₁-C₄-Alkylthio und C₁-C₄-Halogenalkyl;
- ein 5-gliedriger Heteroaromat wie Pyrrolyl, Pyrazolyl, Imidazolyl, Isoxazolyl, Oxazolyl, Isothiazolyl, Thiazolyl, Furanyl und Thienyl, insbesondere Isoxazolyl und Furanyl,
   wobei der 5-gliedrige Heteroaromat unsubstituiert sein oder ein bis drei Substituenten tragen kann, ausgewählt aus einer Gruppe bestehend aus C₁-C₄-Alkyl, C₁-C₄-Alkoxy, C₁-C₄-Alkylthio, C₁-C₄-Halogenalkyl, C₁-C₄-Alkoxy-C₁-C₄-alkyl wie Methoxymethyl, 2-Methoxyethyl, 2-Methoxypropyl, 3-Methoxypropyl, 2-Methoxy-1-methylethyl, Ethoxymethyl, 2-Ethoxyethyl, 2-Ethoxypropyl, 3-Ethoxypropyl, 2-Ethoxy-1-methylethyl und 1-Ethoxy-1-methylethyl, vorzugsweise Methoxyethyl und Ethoxyethyl,
   C₂-C₆-Alkenyl wie Ethenyl und C₃-C₆-Alkenyl, vorzugsweise 1-Methylethen-1-yl,
   C₂-C₆-Alkenyloxy wie Ethenyloxy und C₃-C₆-Alkenyloxy, insbesondere 1-Methylethen-1-yloxy;
- ein 6- oder 7-gliedriger Heterocyclus der
   a) gesättigt sein kann, z.B. Tetrahydropyran-3-yl, Tetrahydropyran-4-yl, Tetrahydrothiopyran-3-yl, Tetrahydrothiopyran-4-yl und Dioxepan-5-yl,
   b) ein- oder zweifach ungesättigt sein kann, z.B. Dihydropyran-3-yl, Dihydropyran-4-yl, Dihydrothiopyran-3-yl und Dihydrothiopyran-4-yl,
   wobei die Heterocyclen unsubstituiert sein oder ein bis drei Substituenten tragen können, ausgewählt aus einer Gruppe bestehend aus Hydroxyl, Halogen, C₁-C₄-Alkyl, C₁-C₄-Alkoxy, C₁-C₄-Alkylthio und C₁-C₄-Halogenalkyl;
   ganz besonders bevorzugt sind Tetrahydropyran-3-yl, Tetrahydropyran-4-yl und Tetrahydrothiopyran-3-yl;
- eine Phenyl- oder Pyridylgruppe, die beide unsubstituiert sein oder ein bis drei Substituenten tragen können, ausgewählt aus einer Gruppe bestehend aus C₁-C₄-Alkyl, C₁-C₄-Alkoxy, C₁-C₄-Alkylthio, C₁-C₄-Halogenalkyl, C₃-C₆-Alkenyloxy, vorzugsweise Prop-2-en-1-yloxy und But-2-en-1-yloxy, C₃-C₆-Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-3-butinyloxy, 2-Methyl-3-butinyloxy, 1-Methyl-2-butinyloxy, 1,1-Dimethyl-2-propinyloxy, 1-Ethyl-2-propinyloxy, 2-Hexinyloxy, 3-Hexinyloxy, 4-Hexinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-2-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy, 2-Ethyl-3butinyloxy und 1-Ethyl-1-methyl-2-propinyloxy, vorzugsweise 2-Propinyloxy und 2-Butinyloxy; einer der drei Substituenten am Phenyl- oder Pyridylring kann auch eine Aminogruppe -NR^{a}R^{b} sein, wobei
   R^{a} für
      Wasserstoff,
      C₁-C₄-Alkyl, vorzugsweise Methyl und Ethyl,
      C₃-C₆-Alkenyl, vorzugsweise Prop-2-en-1-yl und But-2-en-1-yl, C₃-C₆-Alkinyl, vorzugsweise Prop-2-in-1-yl und But-2-in-1-yl, und
   R^{b} für
      Wasserstoff,
      C₁-C₄-Alkyl, vorzugsweise Methyl und Ethyl,
      C₃-C₆-Alkenyl, vorzugsweise Prop-2-en-1-yl und But-2-en1-yl, C₃-C₆-Alkinyl, vorzugsweise Prop-2-in-1-yl und But-2-in-1-yl,
      oder für
      C₁-C₆-Acyl wie Acetyl, Propionyl, n-Butyryl, 2-Methylpropionyl, n-Pentanoyl, 2-Methylbutyryl, 3-Methylbutyryl, 2,2-Dimethylpropionyl, n-Hexanoyl, 2-Methylpentanoyl, 3-Methylpentanoyl, 4-Methylpentanoyl, 2,2-Dimethylbutyryl, 2,3-Dimethylbutyryl, 3,3-Dimethylbutyryl und 2-Ethylbutyryl, vorzugsweise Acetyl und Propionyl, oder Benzoyl, das unsubstituiert sein oder seinerseits ein bis drei Reste tragen kann, ausgewählt aus einer Gruppe bestehend aus Nitro, Cyano, Halogen, vorzugsweise Fluor, Chlor und Brom, C₁-C₄-Alkyl, vorzugsweise Methyl, C₁-C₄-Alkoxy, vorzugsweise Methoxy und Ethoxy, C₁-C₄-Alkylthio, vorzugsweise Methylthio, sowie C₁-C₄-Halogenalkyl, vorzugsweise Trifluormethyl,
stehen.

Als Salze der Verbindungen der Formel I kommen landwirtschaftlich brauchbare Salze, beispielsweise Alkalimetallsalze, insbesondere das Natrium- oder Kaliumsalz, Erdalkalisalze insbesondere das Calcium-, Magnesium- oder Bariumsalz, Mangan-, Kupfer-, Zink- oder Eisensalz sowie Ammonium-, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Unter Estern von C₁-C₁₀-Carbonsäuren sind insbesondere C₁-C₆-Alkylcarbonsäuren wie Methylcarbonsäure (Essigsäure), Ethylcarbonsäure (Propionsäure), Propylcarbonsäure (Buttersäure), 1-Methylethylcarbonsäure (Isobuttersäure), Butylcarbonsäure, 1-Methylpropylcarbonsäure, 2-Methylpropylcarbonsäure, 1,1-Dimethylethylcarbonsäure, Pentylcarbonsäure, 1-Methylbutylcarbonsäure, 2-Methylbutylcarbonsäure, 3-Methylbutylcarbonsäure, 1,1-Dimethylpropylcarbonsäure, 1,2-Dimethylpropylcarbonsäure, 2,2-Dimethylpropylcarbonsäure, 1-Ethylpropylcarbonsäure, Benzoesäure sowie durch Halogen substituierte Benzoesäuren, Hexylcarbonsäure, 1-Methylpentylcarbonsäure, 2-Methylpentylcarbonsäure, 3-Methylpentylcarbonsäure, 4-Methylpentylcarbonsäure, 1,1-Dimethylbutylcarbonsäure, 1,2-Dimethylbutylcarbonsäure, 1,3-Dimethylbutylcarbonsäure, 2,2-Dimethylbutylcarbonsäure, 2,3-Dimethylbutylcarbonsäure, 3,3-Dimethylbutylcarbonsäure, 1-Ethylbutylcarbonsäure, 2-Ethylbutylcarbonsäure, 1,1,2-Trimethylpropylcarbonsäure, 1,2,2-Trimethylpropylcarbonsäure, 1-Ethyl-1-methylpropylcarbonsäure und 1-Ethyl-2-methylpropylcarbonsäure, zu verstehen.

### Herstellungsbeispiele

### 2-[1-[2-[4-(2,4-Dichlorphenoxy)phenoxy]propyloxyimino]propyl]-3-hydroxy-5-(2H-tetrahydropyran-4-yl)-2-cyclohexen-1-on (4.04)

Eine Mischung aus 0,71 g (2,8 mmol) 3-Hydroxy-2-propionyl-5-(2H-tetrahydropyran-4-yl)-2-cyc1ohexen-1-on, 1,2 g (2,8 mmol) 75 %igem O-[2-[4-(2,4-Dichlorphenoxy)phenoxy]propylhydroxylamin und 100 ml Methanol wurde 24 h bei Raumtemperatur gerührt und anschließend in bekannter Weise zum Produkt aufgearbeitet. Ausbeute: 69 %
¹H-NMR (200 MHZ, in CDCl₃): δ [ppm] = 1,15 (t, 3H), 1,20-1,45 (m, 6H), 1,65 (m, 2H), 1,90 (m, 1H), 2,25 (m,2H), 2,60 (m, 2H); 2,85 (m, 2H), 3,35 (m, 2H), 4,00 (m,2H), 4,25 (m,2H), 4,60 (m, 1H), 6,80-7,55 (m, 7H).

### Vorstufe

### O-[2-[4-(2,4-Dichlorphenoxy)phenoxy]propylhydroxylamin

Zu einer Lösung aus 7,0 g (0,043 mol) N-Hydroxyphthalimid, 9,4 g (0,038 mol) Triphenylphosphin und 11,4 g (0,036 mol) 2-[4-(2,4-Dichlorphenoxy)phenoxy]1-propanol (Z. Naturforsch. 37 B; 912 (1982)) in 250 ml Tetrahydrofuran tropfte man langsam 7,5 g (0,043 mol) Azodicarbonsäurediethylester. Nach schwach exothermer Reaktion wurde nach 15 h wie bekannt zum Zwischenprodukt aufgearbeitet. 16,3 g N-[2-[4-(2,4-Dichlorphenoxy)phenoxy]pro-poxy]phthalimid.

Dieses Phthalimid-Rohprodukt wurde nachfolgend langsam mit 200 ml Ethanolamin versetzt. Nach 3,5 h bei 60°C goß man die Reaktionsmischung in Eiswasser, extrahierte mit Methylenchlorid, wusch die vereinten organischen Phasen mit Wasser, trocknete über Natriumsulfat und engte unter reduziertem Druck ein. Ausbeute: 9,7 g (62 % korr.) 75 %iges Hydroxylamin (1.03)
¹H-NMR (200 MHZ, in CDCl₃): δ [ppm] = 1,30 (d, 3H), 3,80 (m, 2H), 4,65 (m,1H), 5,55 (bs, 1H), 6,80-7,50 (m, 7H).

In der folgenden Tabelle 1 sind neue Hydroxylamine III aufgelistet. Die Tabellen 2 bis 17 enthalten erfindungsgemäße Cyclohexenonoximether I.

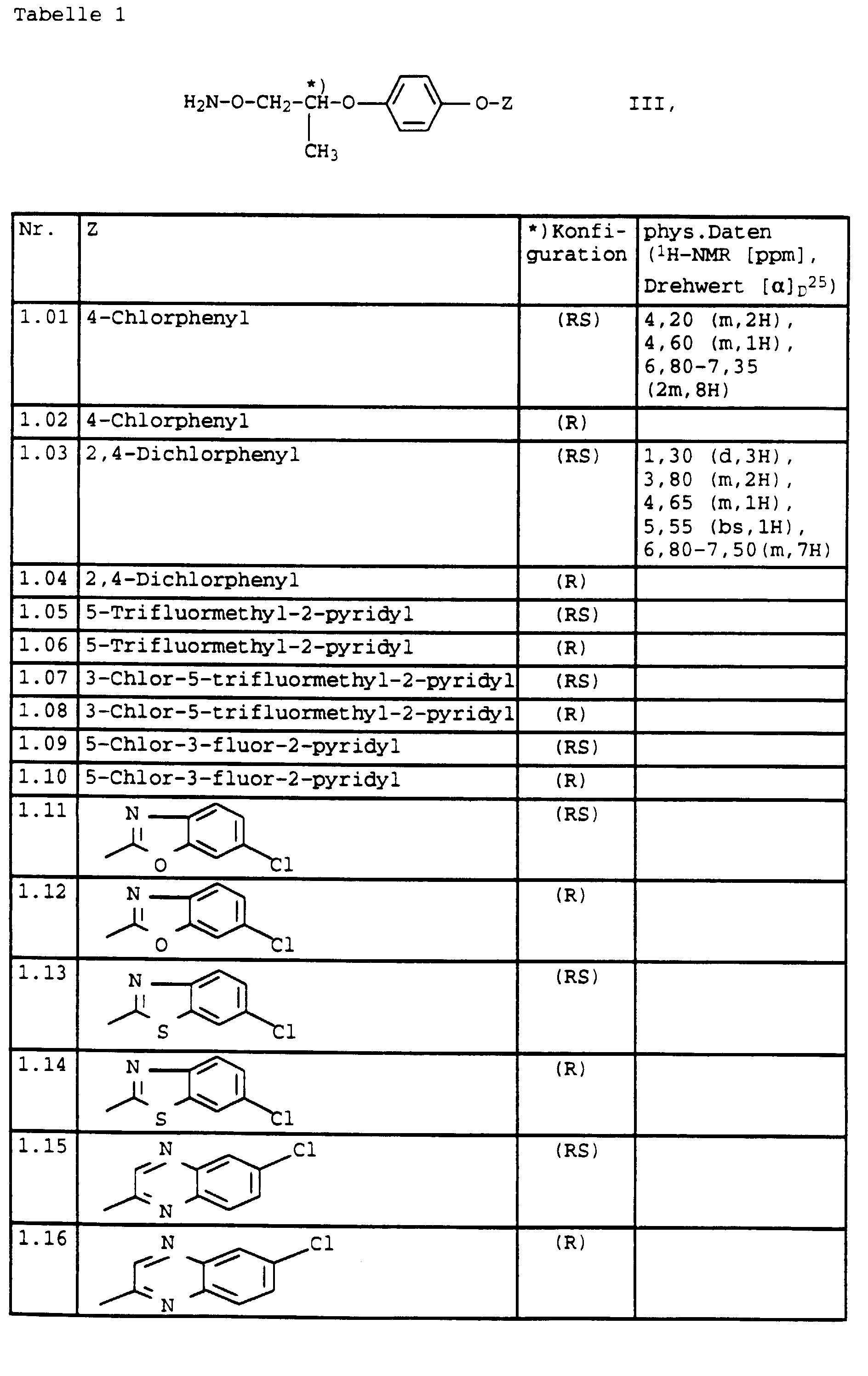

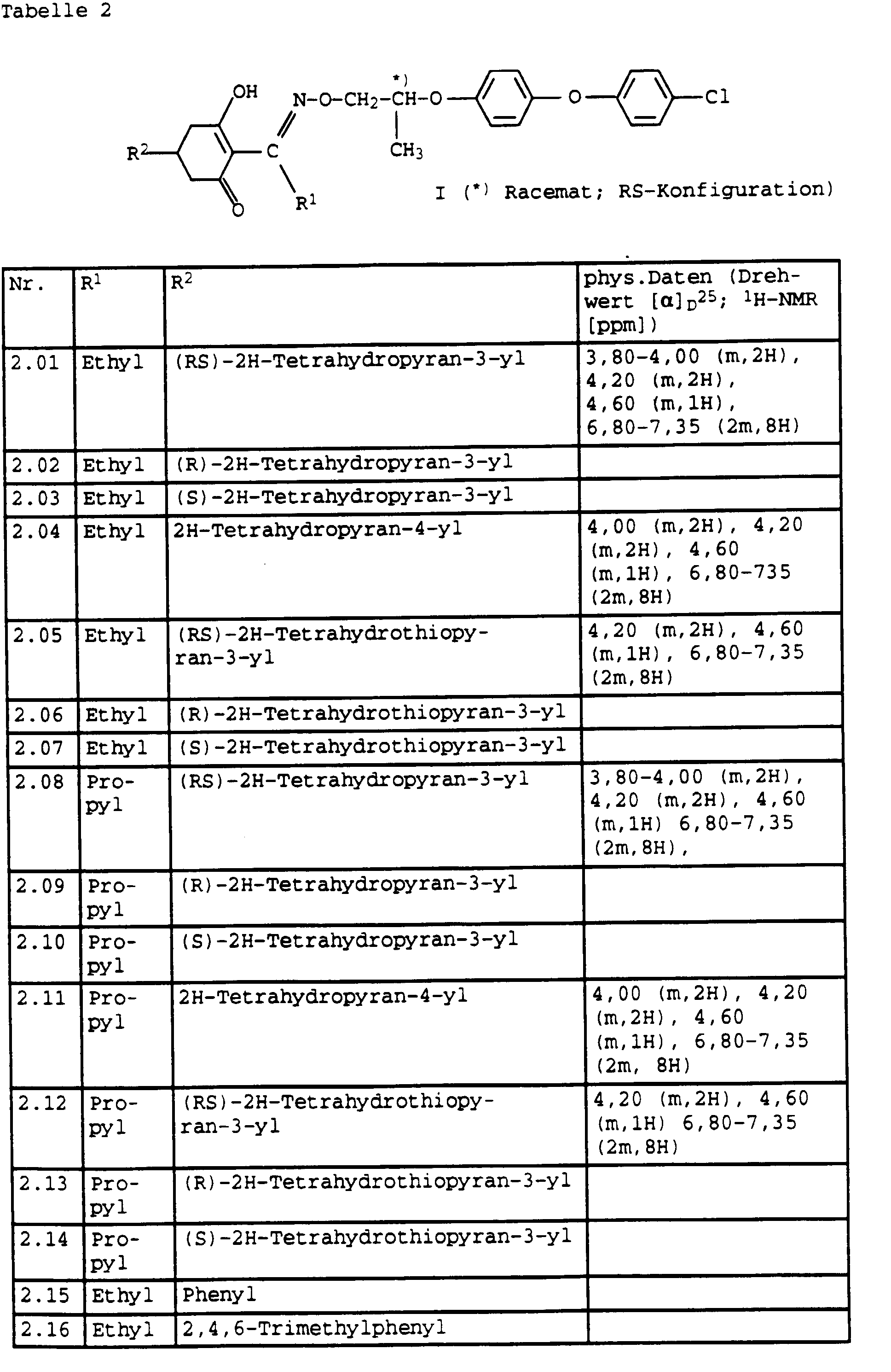

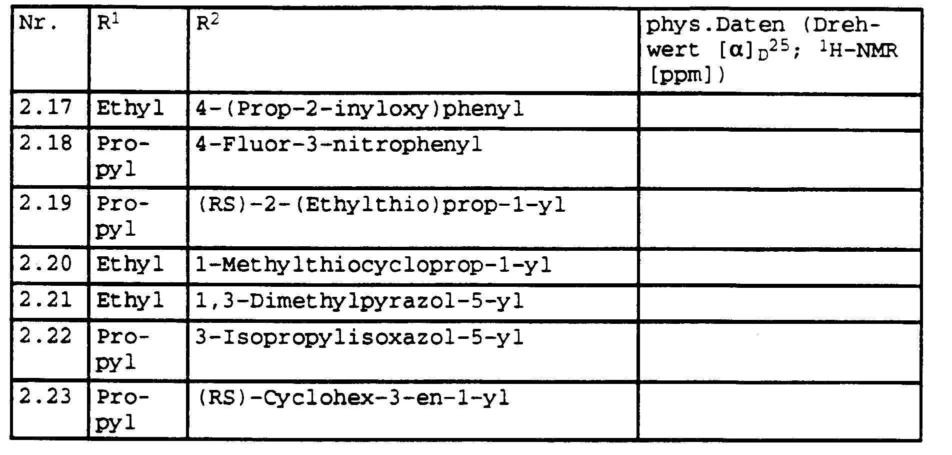

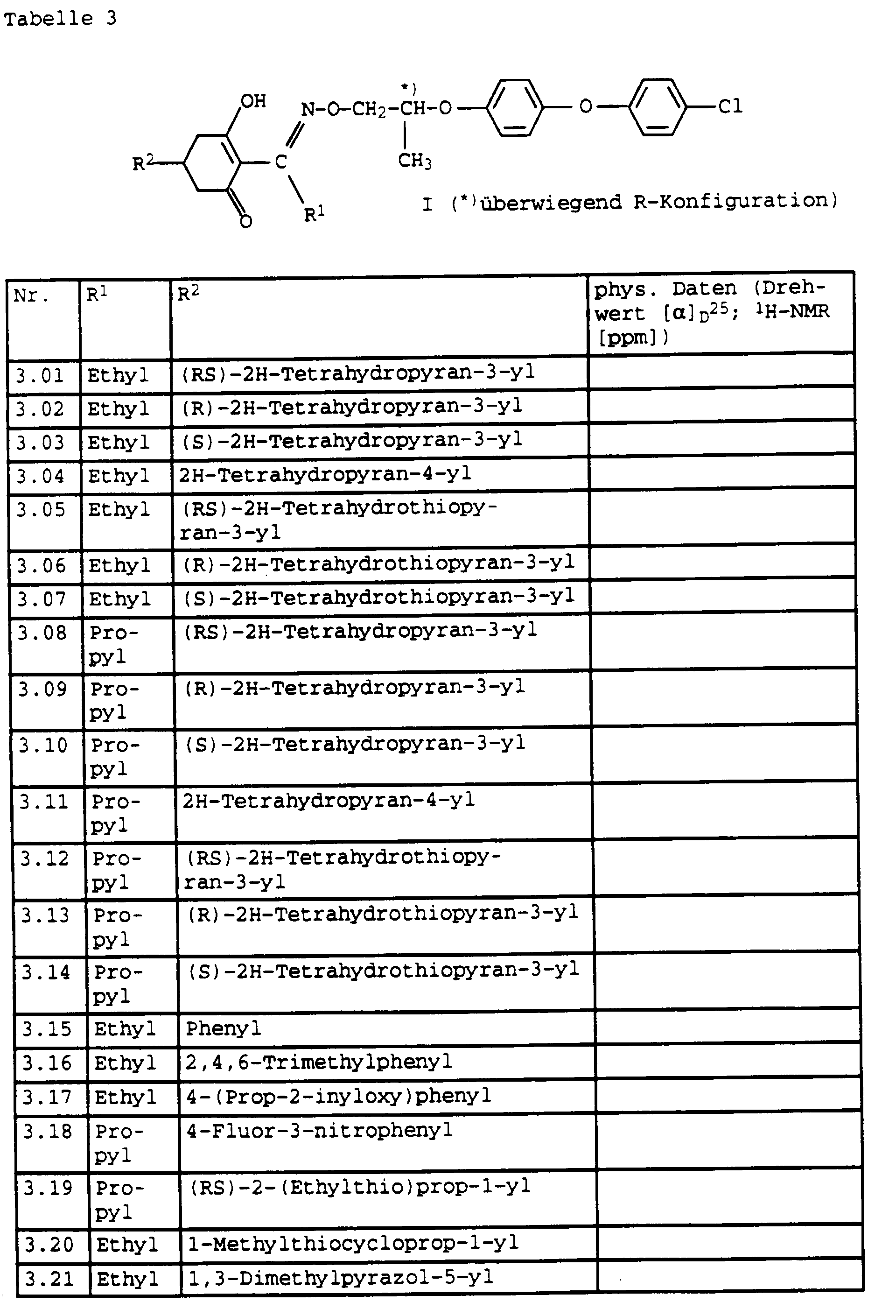

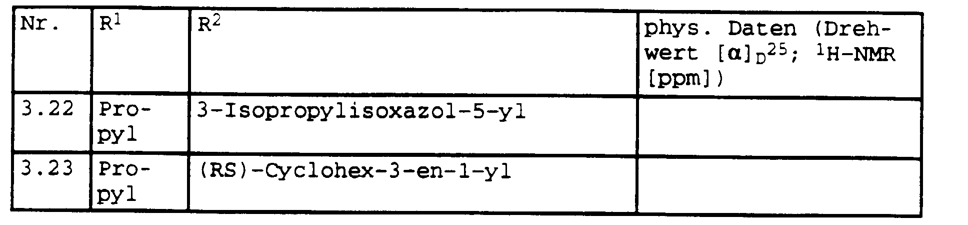

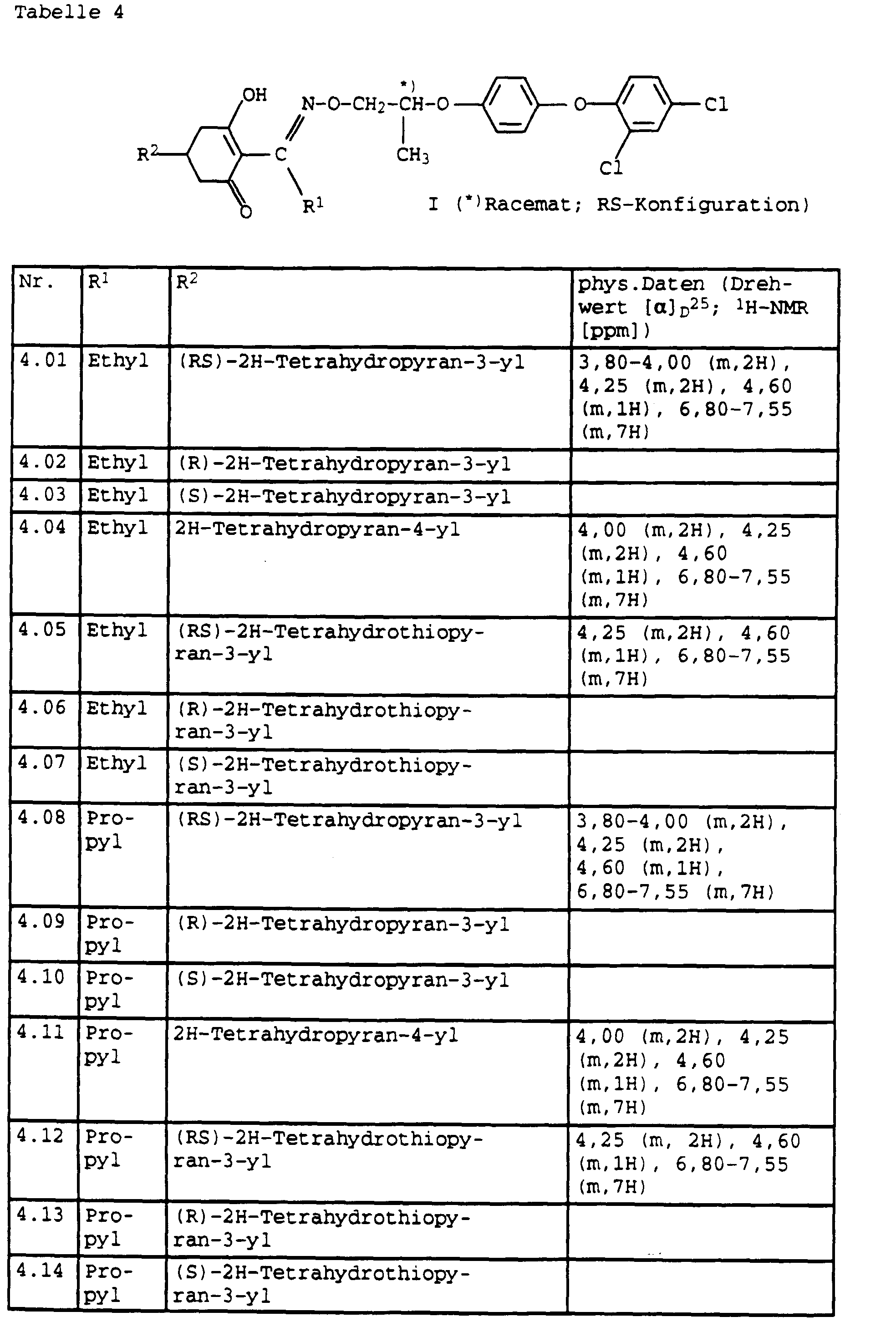

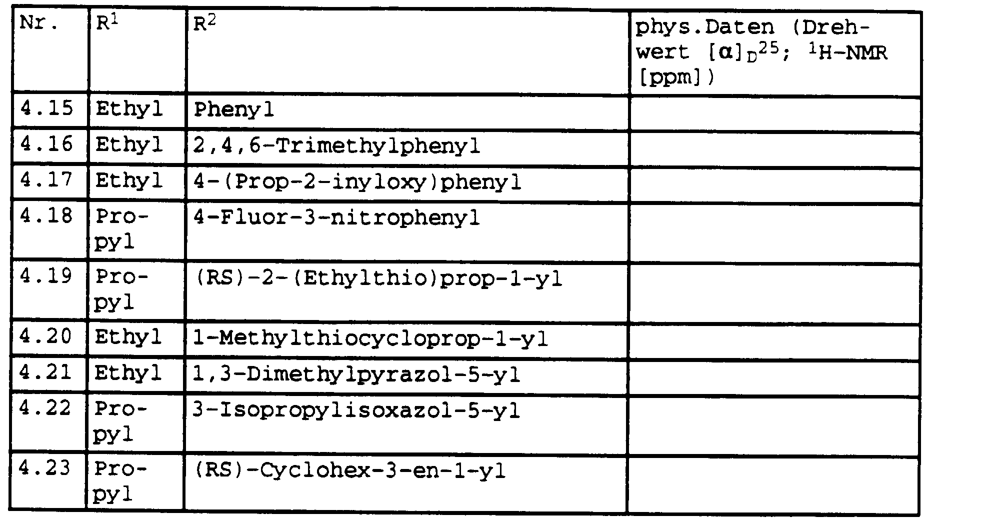

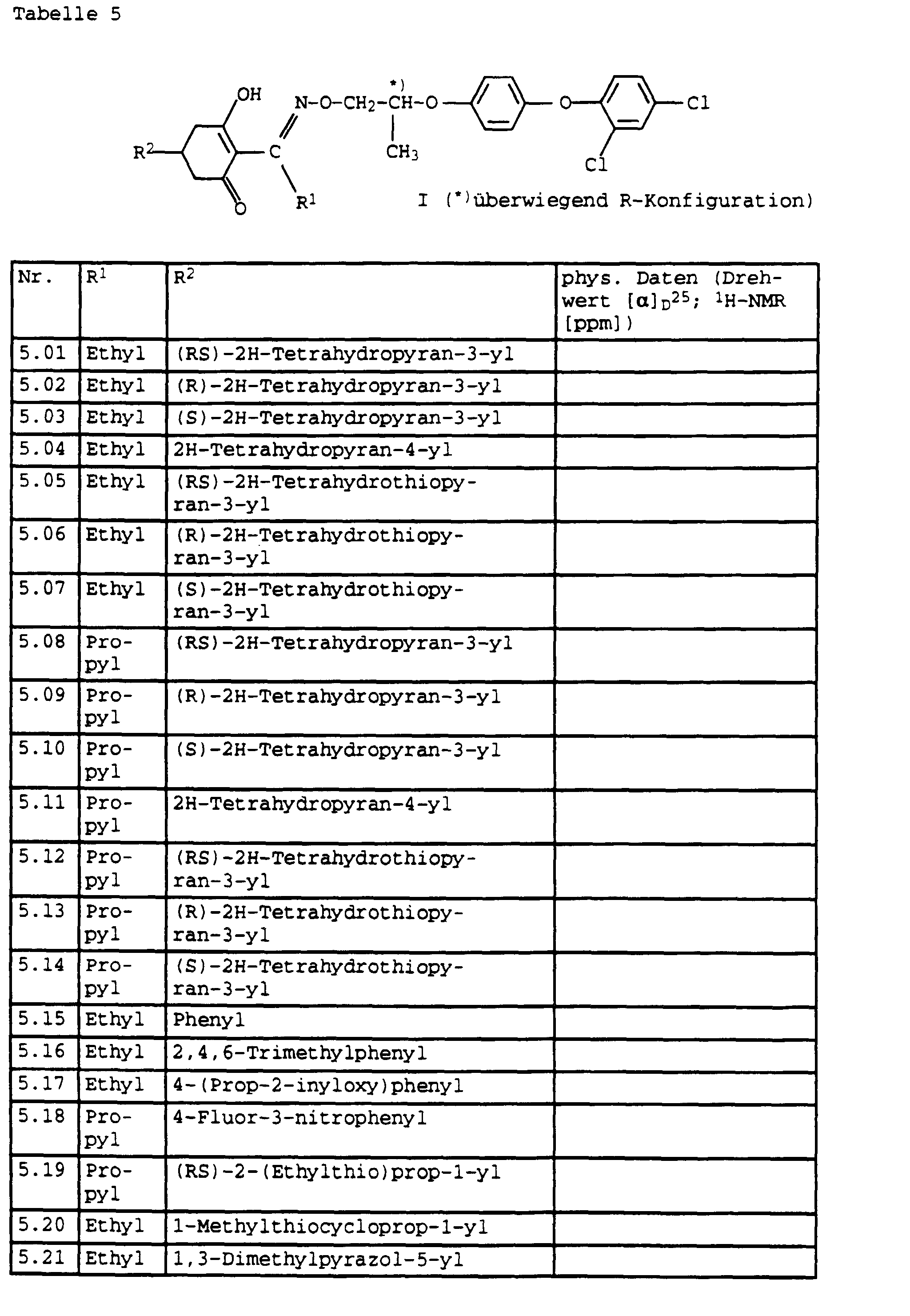

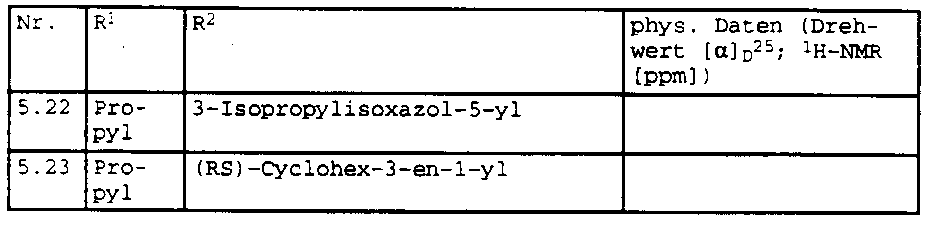

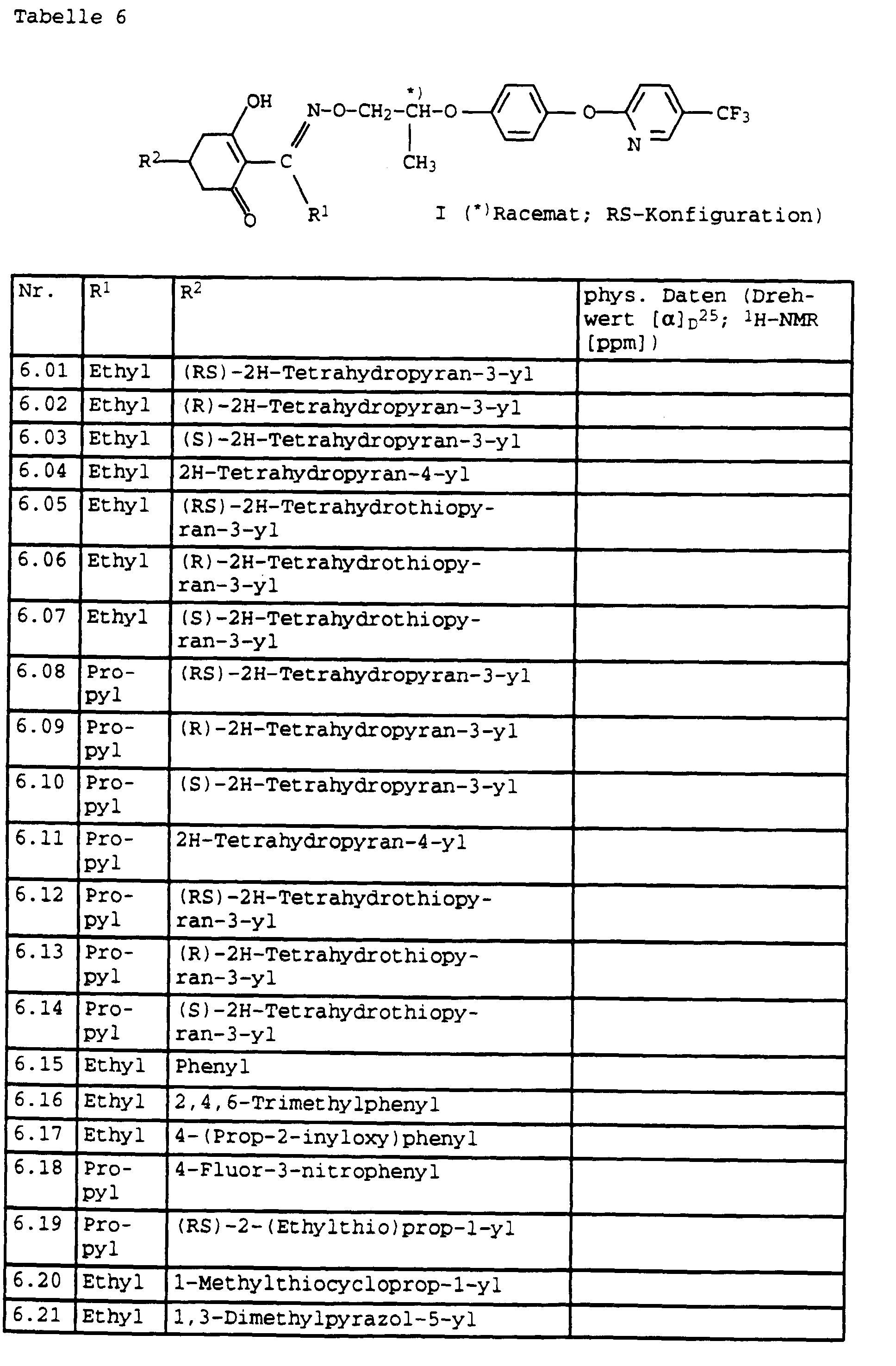

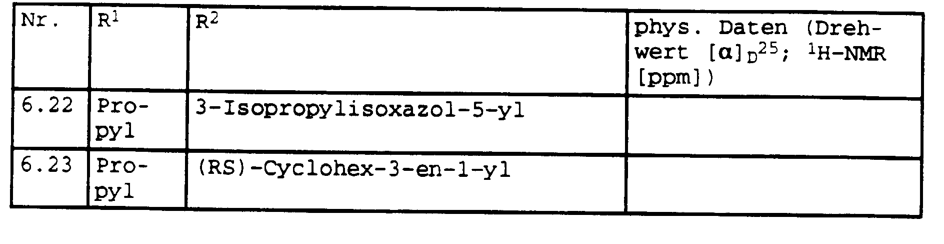

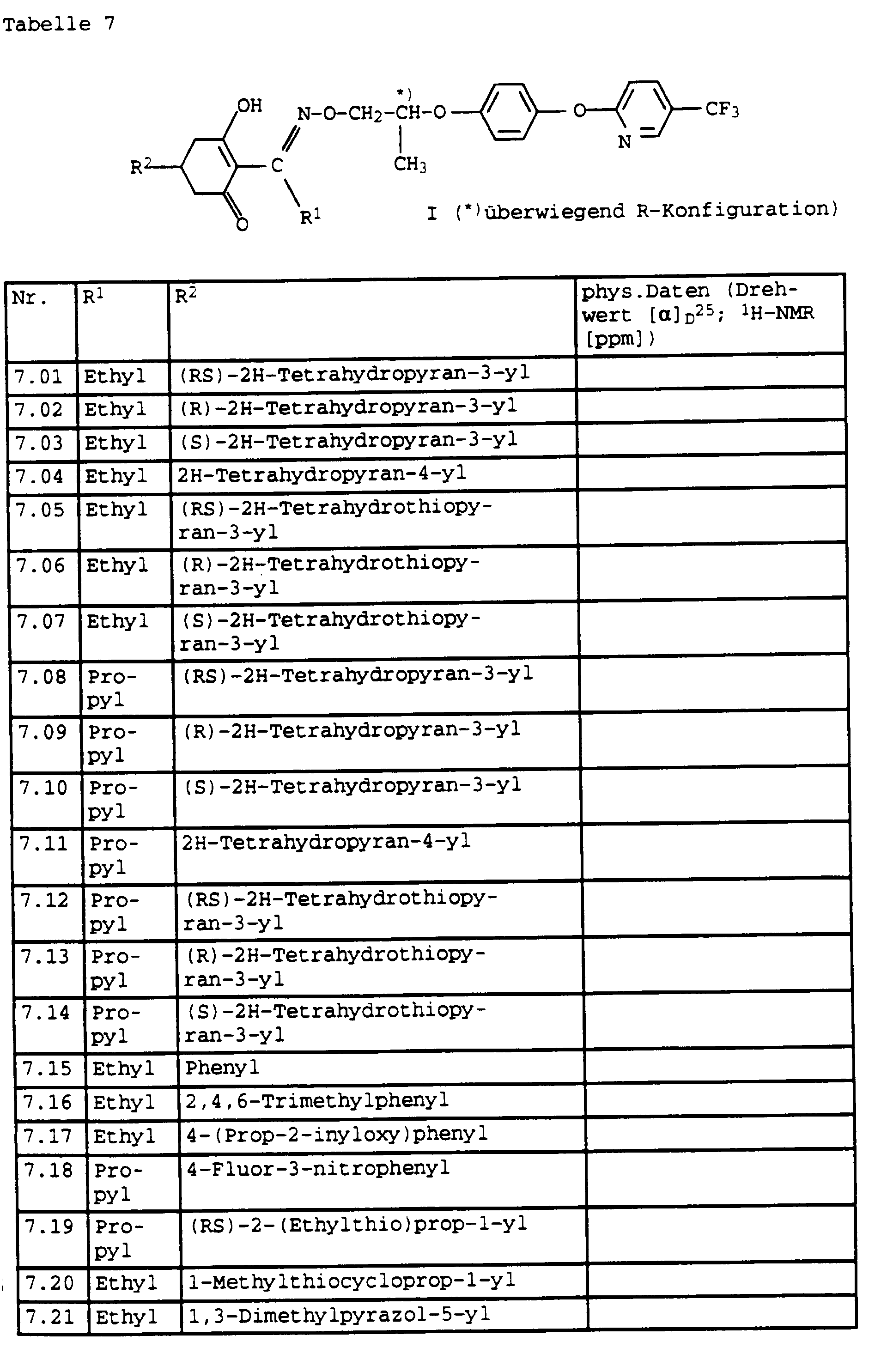

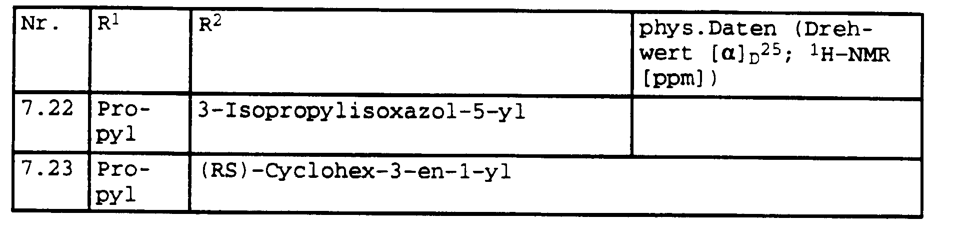

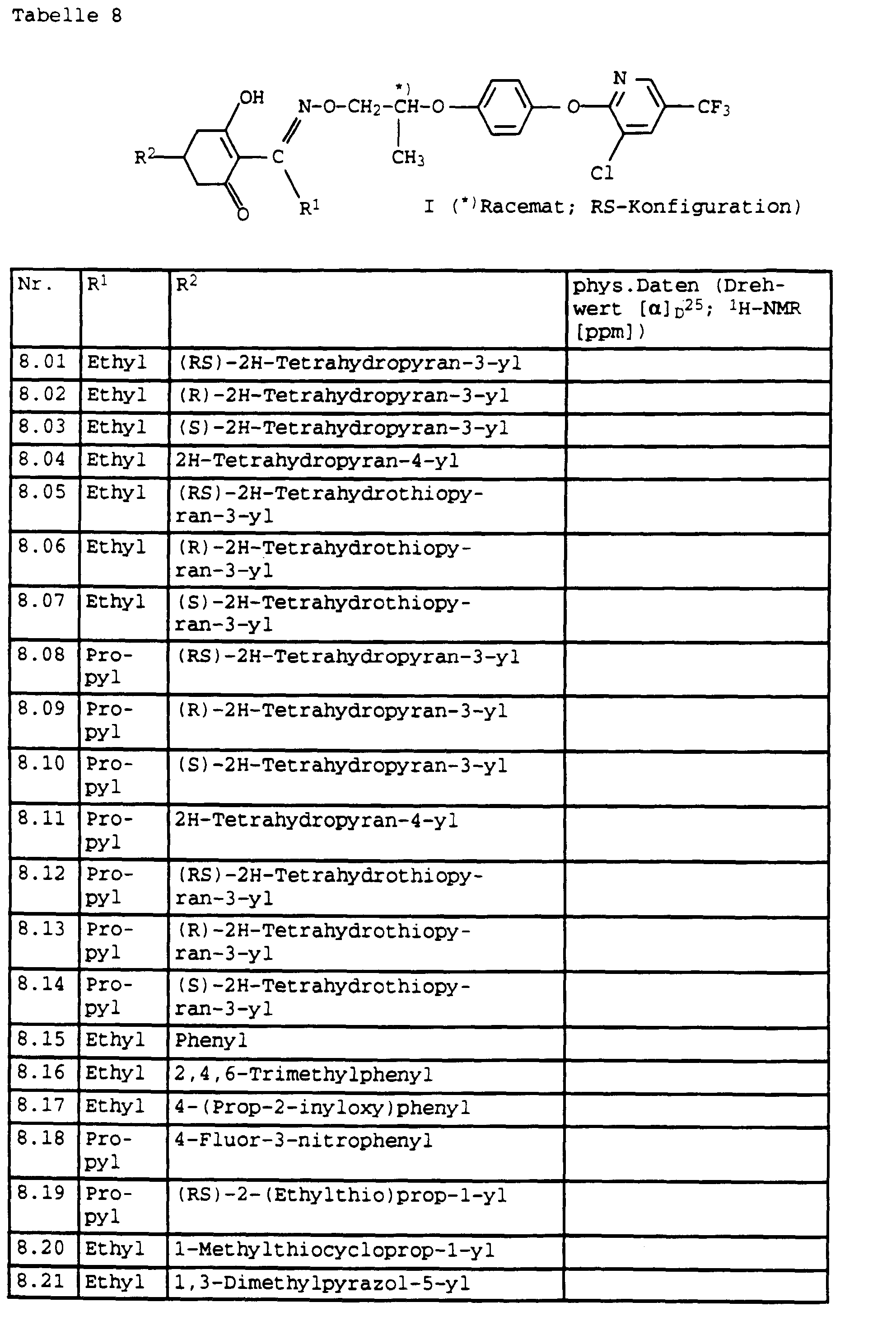

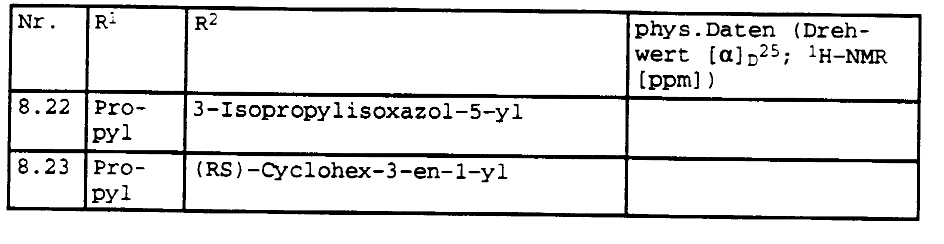

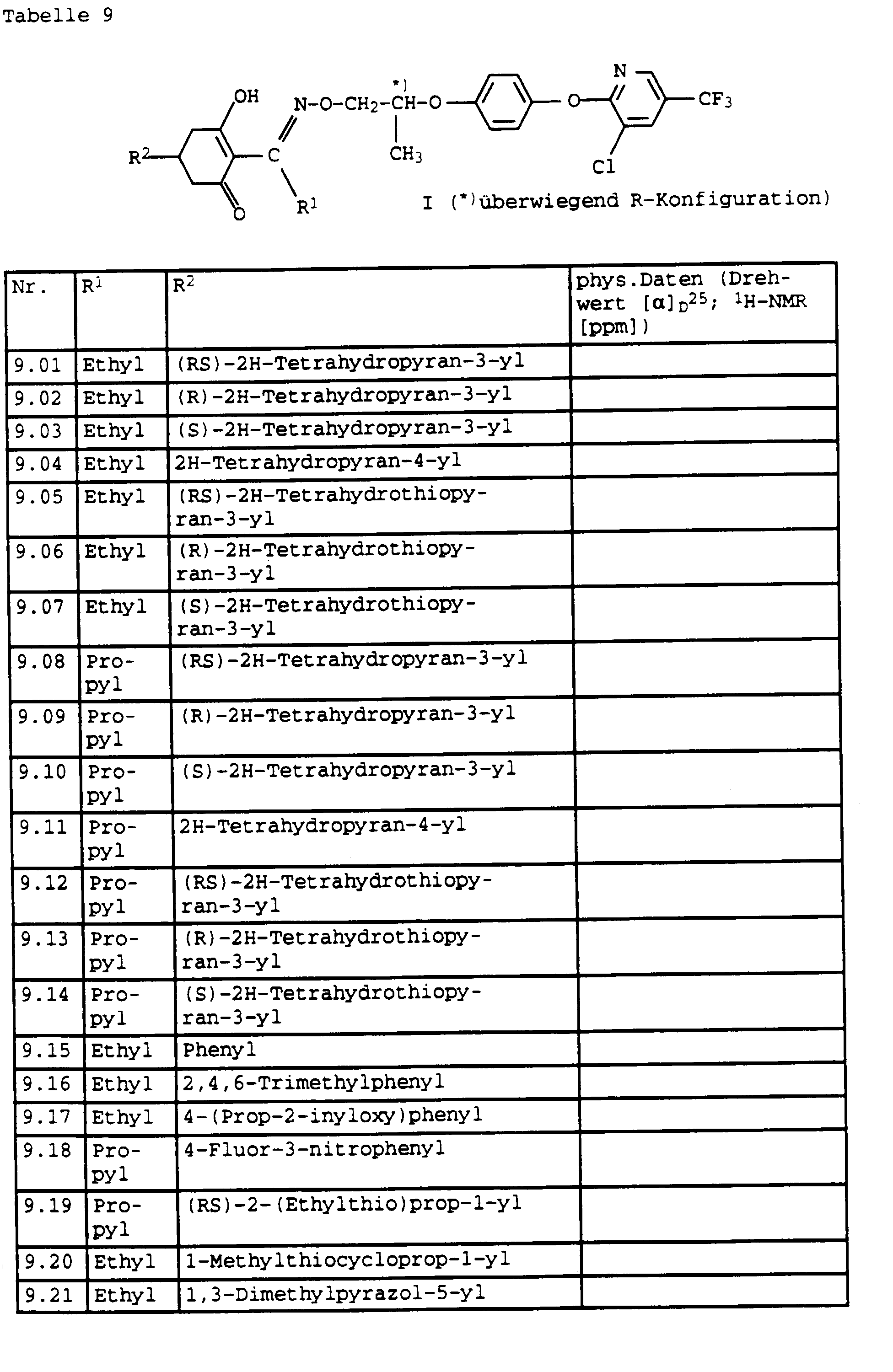

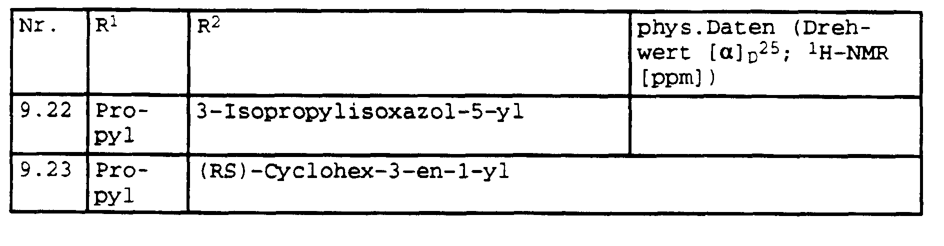

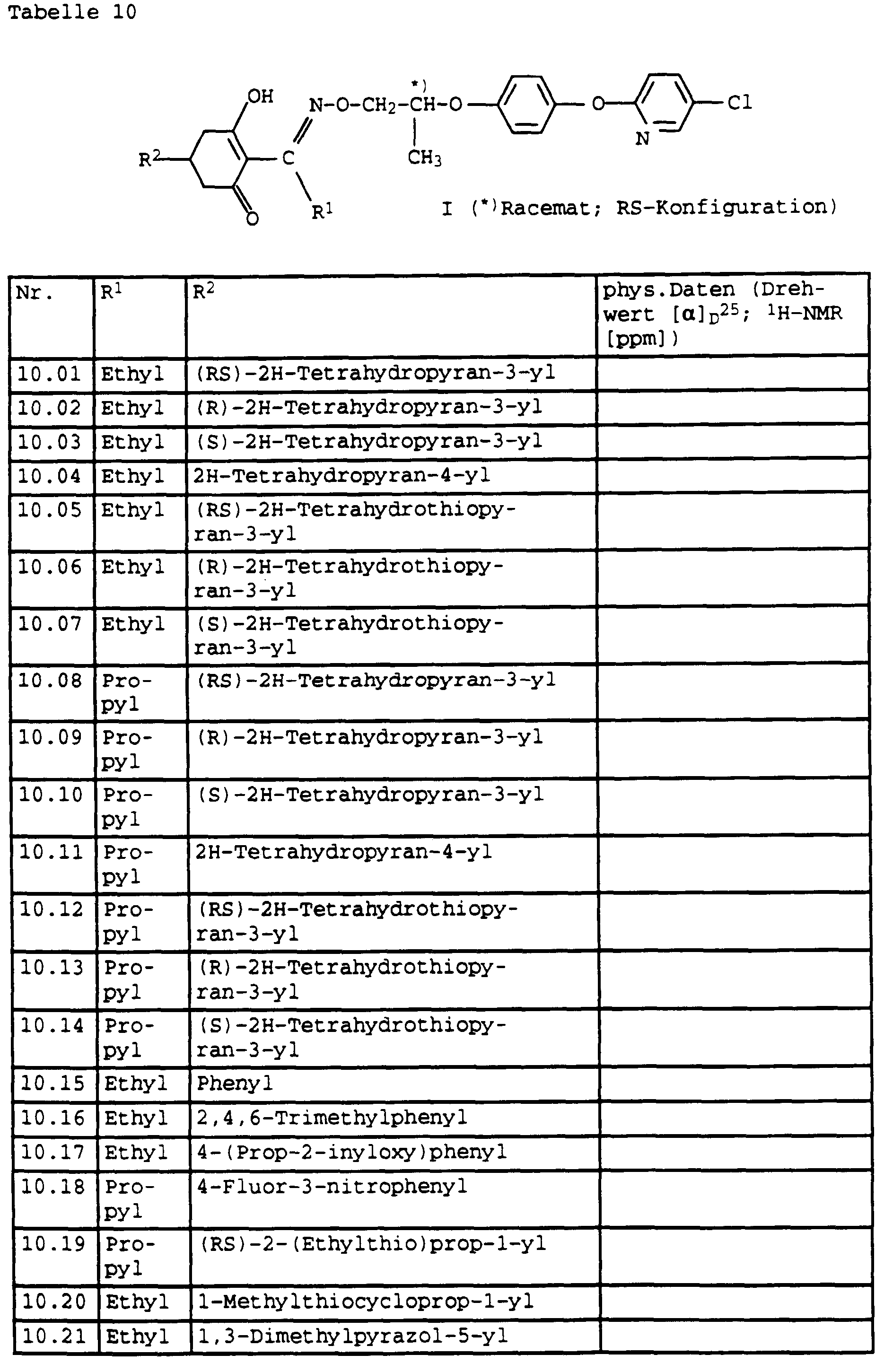

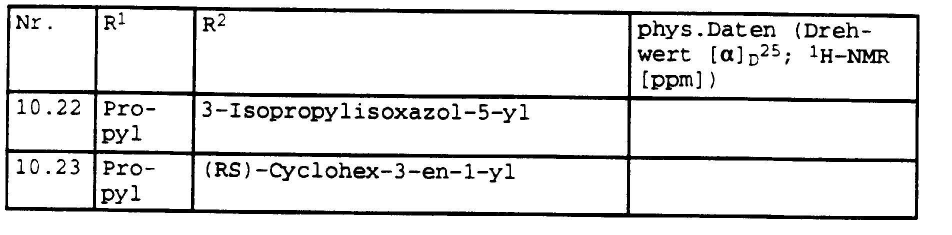

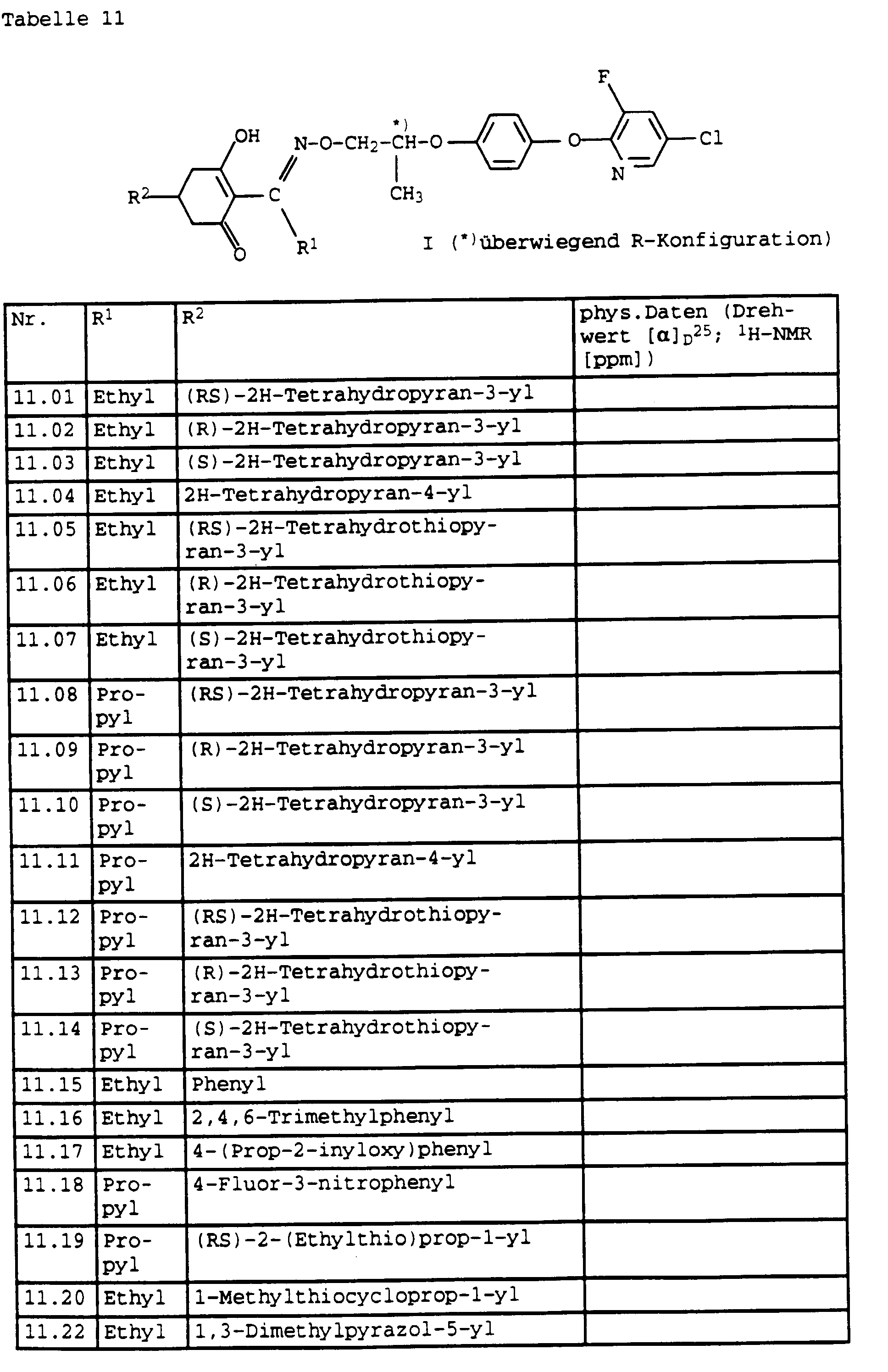

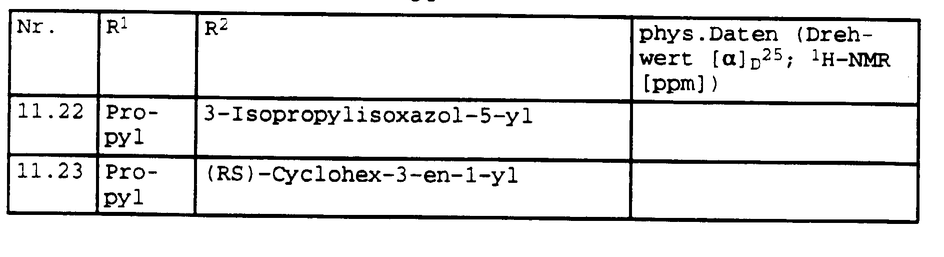

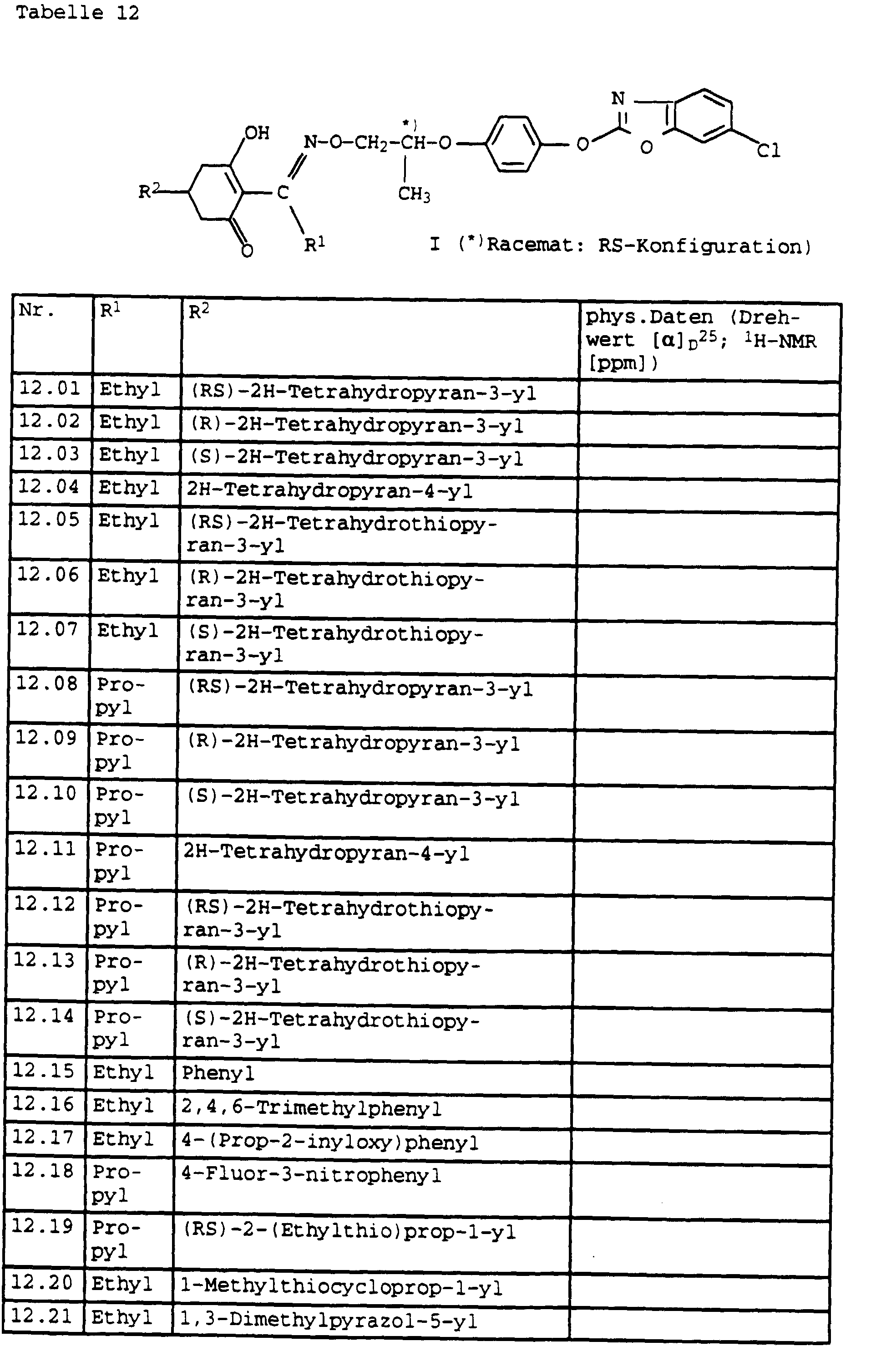

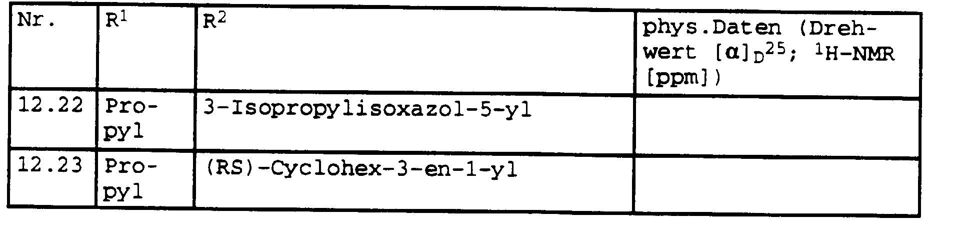

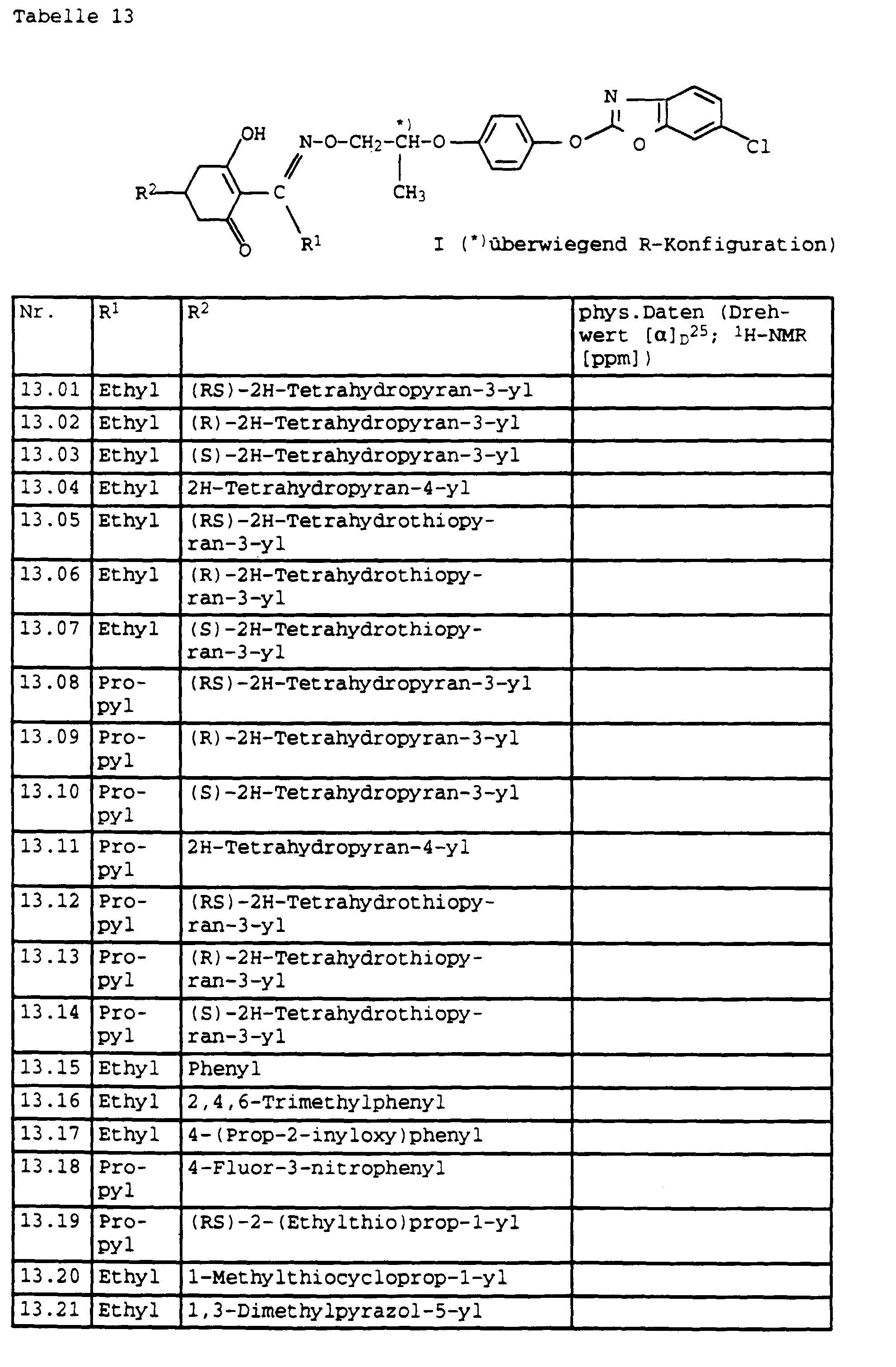

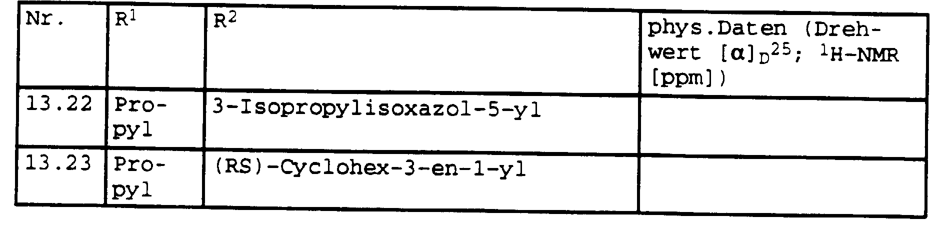

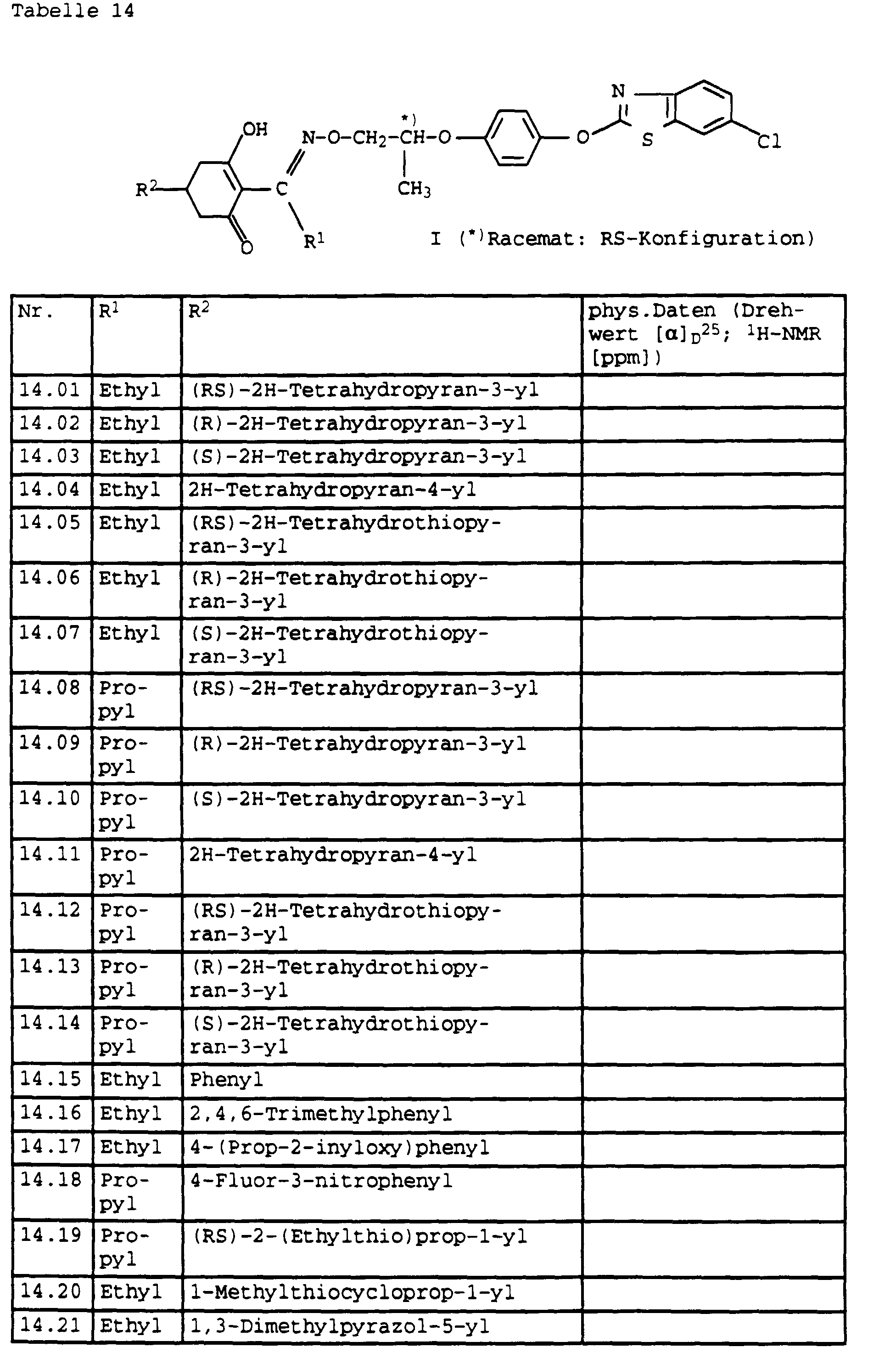

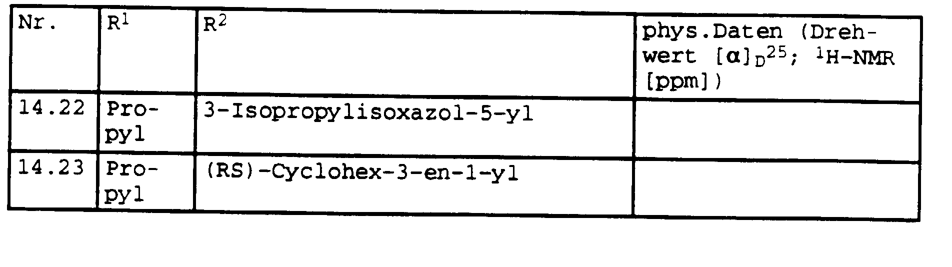

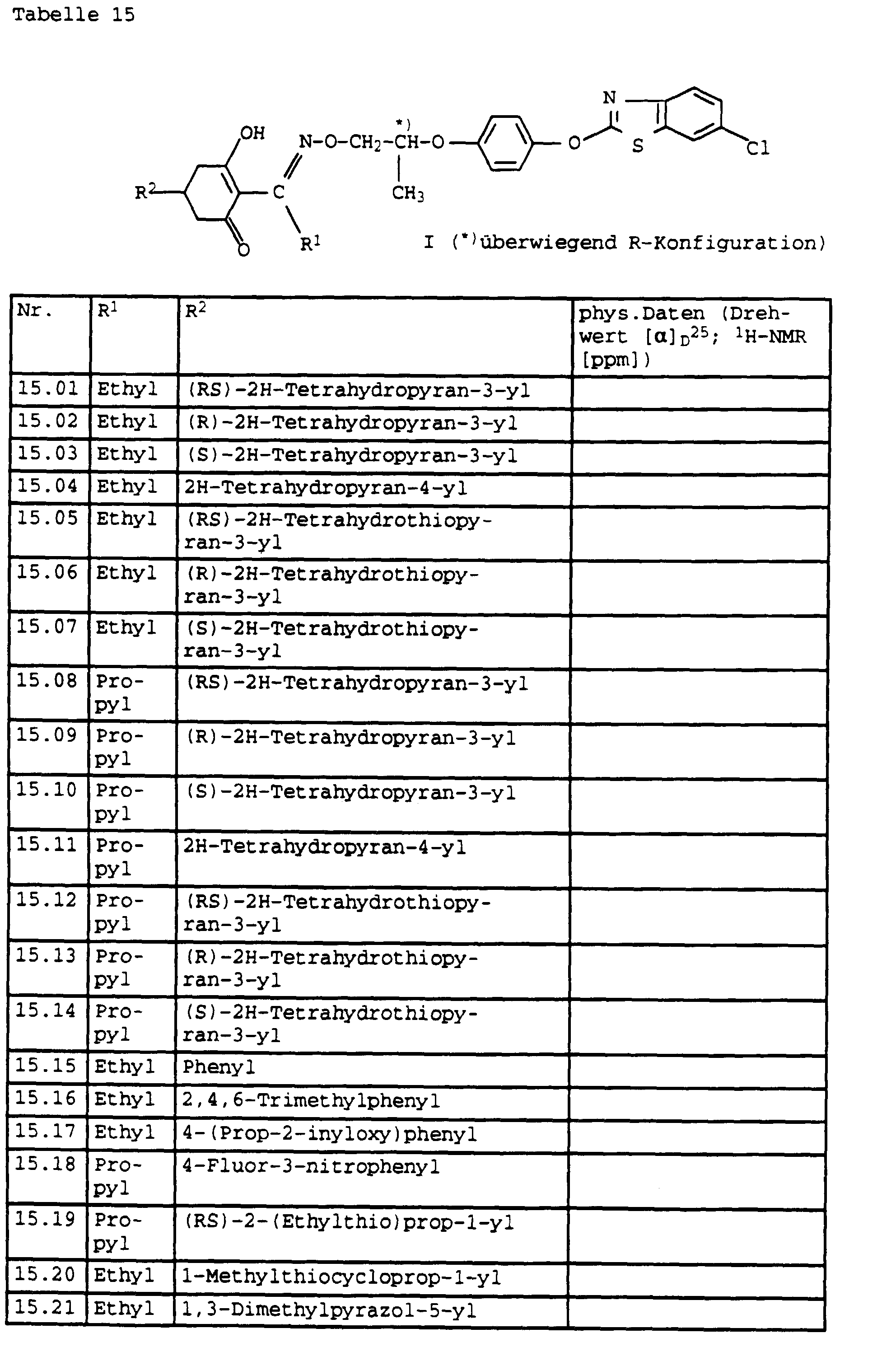

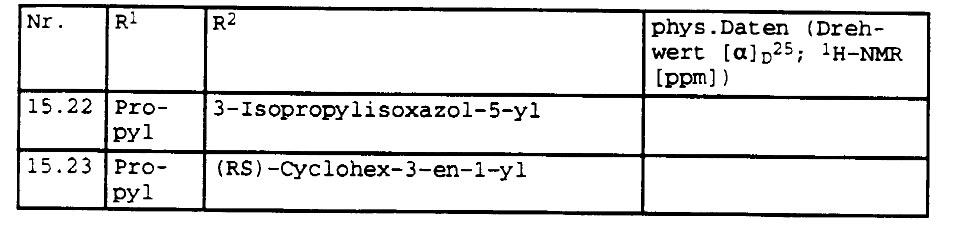

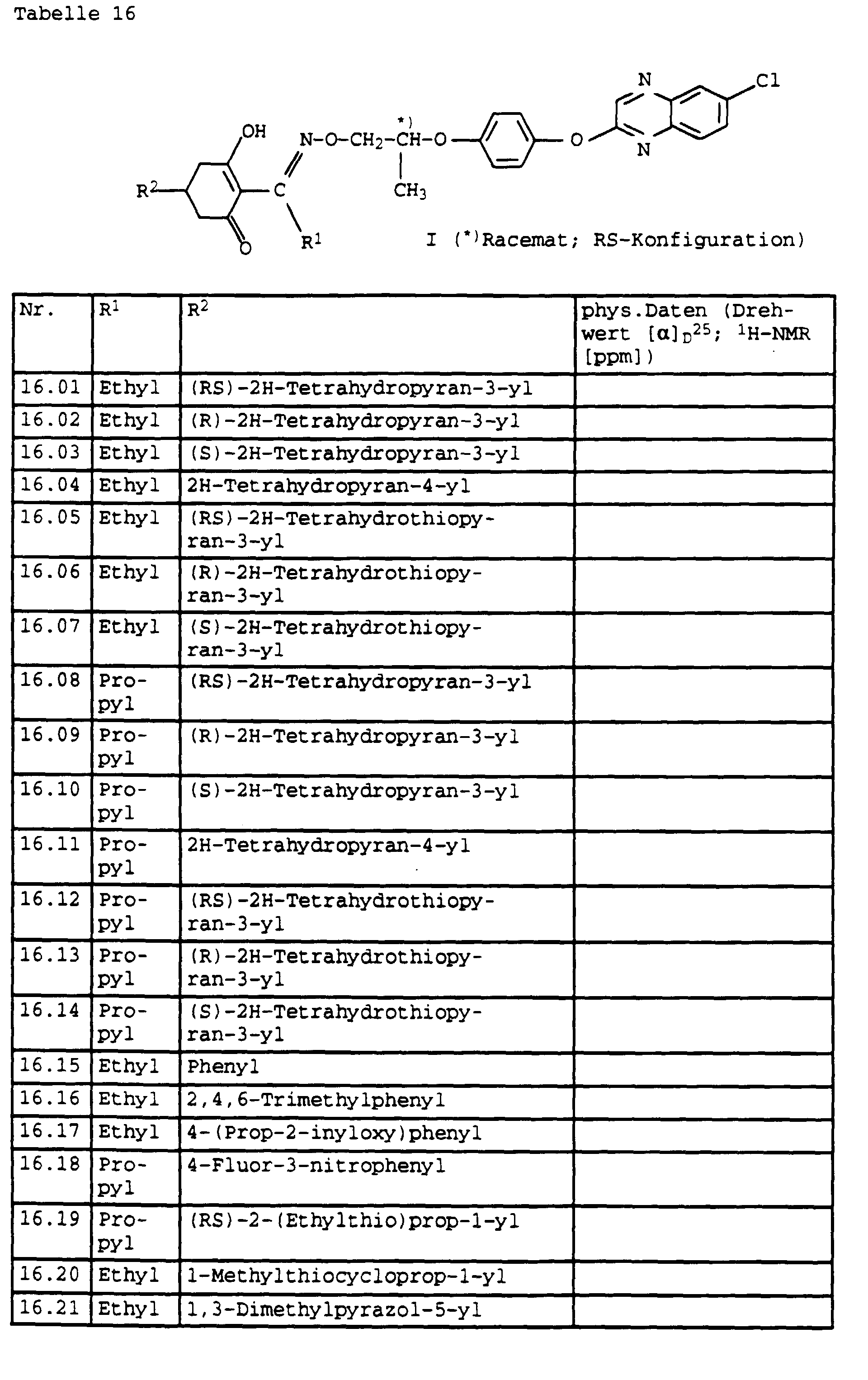

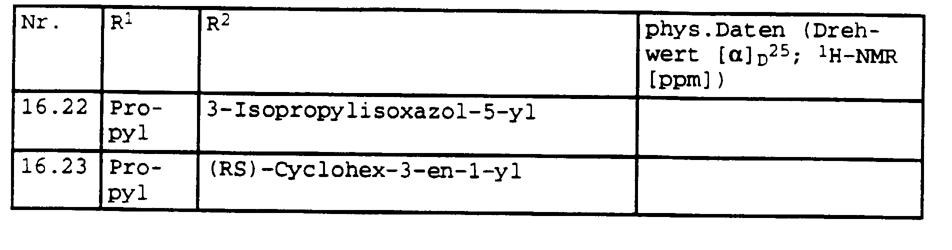

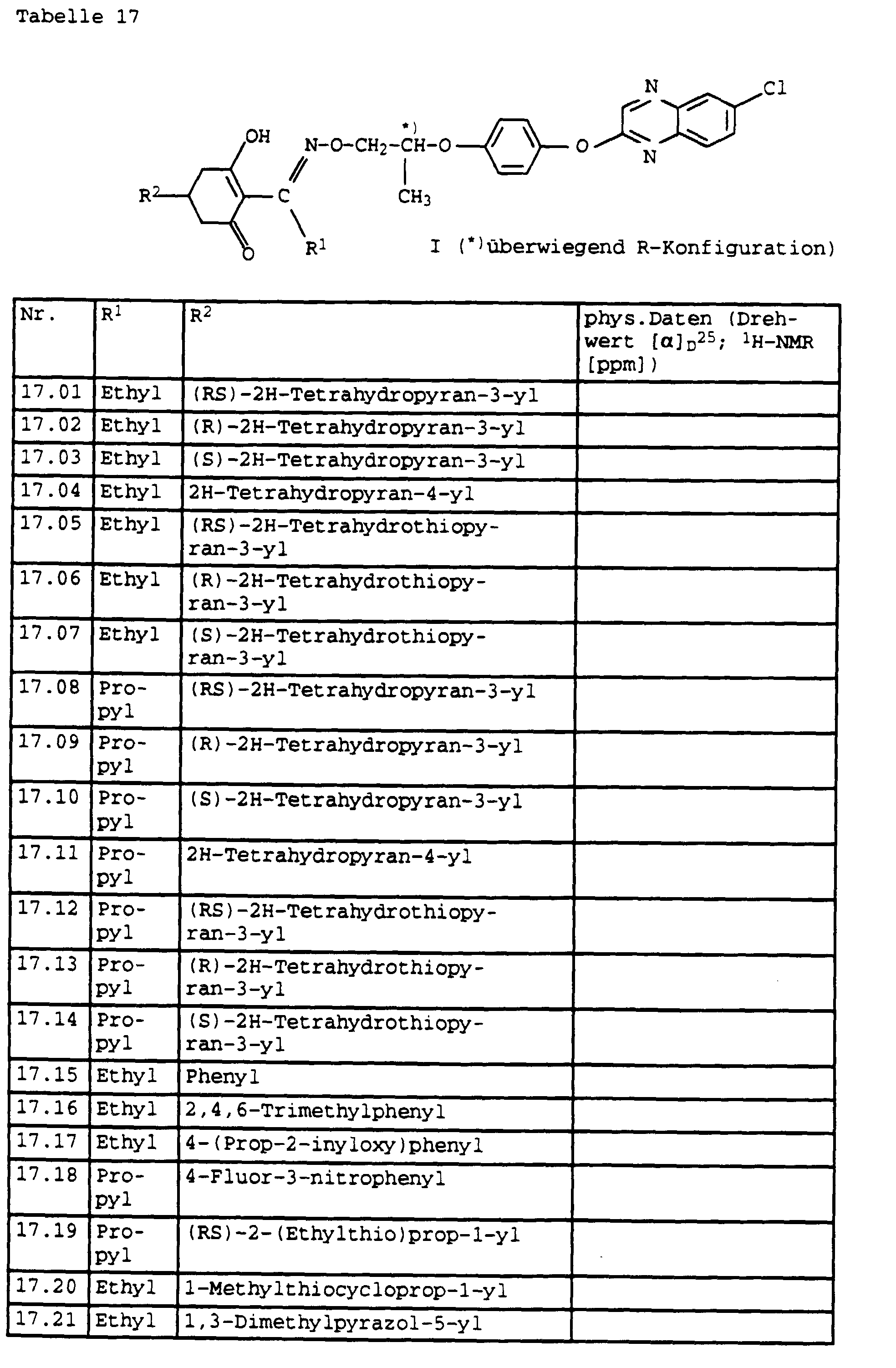

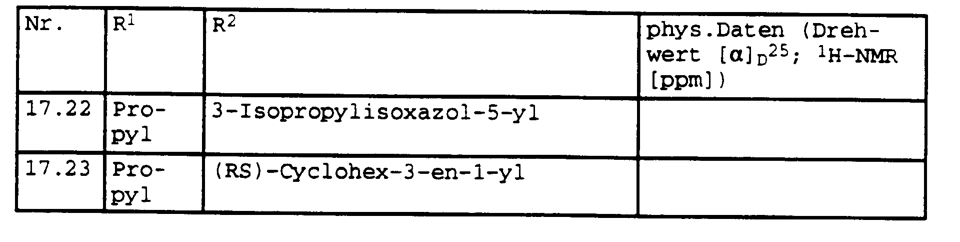

Die optisch aktiven Cyclohexenonoximether I eignen sich, sowohl als Isomerengemische als auch in Form der reinen Isomeren, als Herbizide, insbesondere zur Bekämpfung von Pflanzenarten aus der Familie der Gräser (Gramineen). Im allgemeinen sind sie verträglich und somit selektiv in breitblättrigen Kulturen sowie in monokotylen (einkeimblättrigen) Gewächsen, welche nicht zu den Gramineen zählen. Einige der erfindungsgemäßen Cyclohexenonoximether I sind auch zur selektiven Bekämpfung von unerwünschten Gräsern in Gramineenkulturen geeignet.

Die optisch aktiven Cyclohexenonoximether I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Mischungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdhlkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

### Beispiele für solche Zubereitungen sind:

I. eine Lösung aus 90 Gew.-Teilen der Verbindung Nr. 2.01 und 10 Gew.-Teilen N-Methyl-α-pyrrolidon, die zur Anwendung in Form kleinster Tropfen geeignet ist;
II. eine Mischung aus 20 Gew.-Teilen der Verbindung Nr. 2.03, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes und 40 Mol Ethylenoxid an 1 Mol Ricinusöl. Durch feines Verteilen des Gemisches in 100 000 Gew.-Teilen Wasser erhält man eine Dispersion, die 0,02 Gew.-% des Wirkstoffs enthält.
III. eine wäßrige Dispersion aus 20 Gew.-Teilen der Verbindung Nr. 2.05, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl. Die Mischung dieser Dispersion mit 100 000 Gewichtsteilen Wasser enthält 0,02 Gew.-% des Wirkstoffes.
IV. eine wäßrige Dispersion aus 20 Gew.-Teilen der Verbindung Nr. 2.07, 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl. Die Mischung dieser Dispersion mit 100 000 Gew.-Teilen Wasser enthält 0,02 % des Wirkstoffes;
V. eine in einer Hammermühle vermahlene Mischung aus 80 Gew.-Teilen der Verbindung Nr. 2.09, 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel. Durch feines Verteilen der Mischung in 20 000 Gew.-Teilen Wasser erhält man eine Spritzbrühe, die 0,1 Gew.-% des Wirkstoffs enthält;
VI. eine innige Mischung aus 3 Gew.-Teilen der Verbindung Nr. 2.11 und 97 Gew.-Teilen feinteiligem Kaolin. Dieses Stäubemittel enthält 3 Gew.-% Wirkstoff;
VII. eine innige Mischung aus 30 Gew.-Teilen der Verbindung Nr. 2.13, 92 Gew.-Teilen pulverförmigem Kieselsäuregel und 8 Gew.-Teilen Paraffinöl, das auf die Oberfläche dieses Kieselsäuregels gesprüht wurde. Diese Aufbereitung gibt dem Wirkstoff eine gute Haftfähigkeit;
VIII. eine stabile wäßrige Dispersion aus 40 Gew.-Teilen der Verbindung Nr. 2.15, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung Nr. 3.01, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäureharnstoff-formaldehyd-Kondensates und 68 Gew.-Teilen eines paraffinischen Mineralöls;
X. eine in einer Hammermühle vermahlene Mischung aus 10 Gew.-Teilen der Verbindung Nr. 4.03, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgerate so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1 kg/ha aktive Substanz (a.S.).

In Anbetracht der Vielseitigkeit der Applikationsmethoden können die Cyclohexenonoximether I bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rüben |
| Camellia sinensis | Teestrauch |
| Carthamus tinctorius | Saflor-Färberdistel |
| Carya illinoinensis | Pekannußbaum |
| Citrus limon | Zitrone |
| Citrus sinensis | Apfelsine, Orange |

| Botanischer Name | Deutscher Name |
|---|---|
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohnen |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |

| Botanischer Name | Deutscher Name |
|---|---|
| Vitis vinifera | Weinrebe |
| Zea mays | Mais |

Zur Verbreiterung des Wirkungsspektrums und zur Erzielung synergistischer Effekte können die Cyclohexenonoximether I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmangeln eingesetzt werden. Es können auch nichtphytotoxische Öle und Ölkonzentrate zugesetzt werden.

### Anwendungsbeispiele

Die herbizide Wirkung der ungesättigten Cyclohexenonoximether der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen bereits in den Versuchsgefäßen angezogen oder einige Tage vorher ir die Versuchsgefäße verpflanzt. Die Applikation der in Wasser suspendierten oder emulgierten Wirkstoffe erfolgte je nach Wuchsform erst bei einer Wuchshöhe von 3 bis 15 cm. Die Aufwandmenge für die Nachauflaufbehandlung betrug 0,25 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

## Patentansprüche

1. Mischungen aus optisch aktiven Cyclohexenonoximethern mit R- und S-Konfiguration im Oximetherteil der allgemeinen Formel I 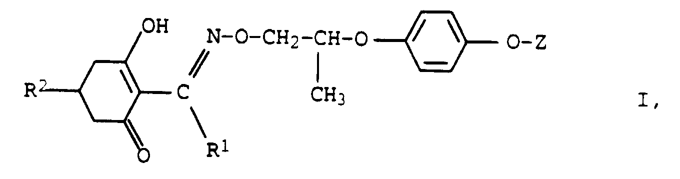 in der die Variablen folgende Bedeutung haben:
R¹ eine C₁-C₆-Alkylgruppe;
Z eine der folgenden Gruppen: 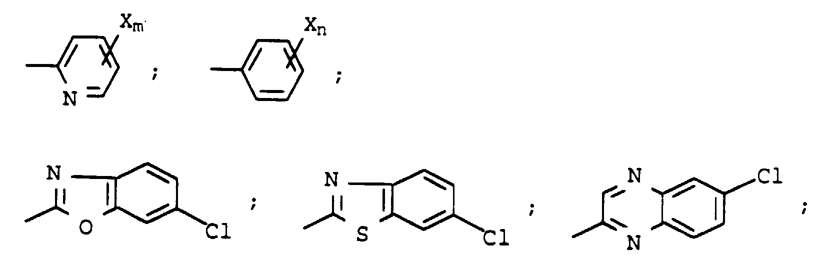
X Halogen, C₁-C₄-Halogenalkyl;
m 0 bis 3 oder 1 bis 4 für den Fall, daß alle X Halogen bedeuten;
n 0 bis 3 oder 1 bis 5 für den Fall, daß alle X Halogen bedeuten;
R² eine C₁-C₄-Alkoxy-C₁-C₆-alkyl- oder C₁-C₄-Alkylthio- C₁-C₆-alkylgruppe;
eine C₃-C₇-Cycloalkylgruppe oder eine C₅-C₇-Cycloalkenylgruppe, wobei diese Gruppen gewünschtenfalls ein bis drei Substituenten tragen können, ausgewählt aus einer Gruppe bestehend aus C₁-C₄-Alkyl, C₁-C₄-Alkoxy, C₁-C₄-Alkylthio, C₁-C₄-Halogenalkyl, Hydroxyl und Halogen;
ein 5-gliedriger gesättigter Heterocyclus, der ein oder zwei Sauerstoff- und/oder Schwefelatome als Heteroatome enthält und der gewünschtenfalls noch ein bis drei Substituenten tragen kann, ausgewählt aus einer Gruppe bestehend aus C₁-C₄-Alkyl, C₁-C₄-Alkoxy, C₁-C₄-Alkylthio und C₁-C₄-Halogenalkyl;
ein 6- oder 7-gliedriger gesättigter oder ein- oder zweifach ungesättigter Heterocyclus, der ein- oder zwei Sauerstoff- oder Schwefelatome oder ein Sauerstoff- und ein Schwefelatom als Heteroatome enthält,
wobei der Heterocyclus gewünschtenfalls noch ein bis drei Substituenten tragen kann, ausgewählt aus einer Gruppe bestehend aus Hydroxyl, Halogen, C₁-C₄-Alkyl, C₁-C₄-Alkoxy, C₁-C₄-Alkylthio und C₁-C₄-Halogenalkyl;
ein 5-gliedriger Heteroaromat, enthaltend ein bis drei Heteroatome, ausgewählt aus einer Gruppe bestehend aus ein oder zwei Stickstoffatomen und ein Sauerstoff- oder Schwefelatom,
wobei der Heteroaromat gewünschtenfalls noch ein bis drei Substituenten tragen kann, ausgewählt aus einer Gruppe bestehend aus Halogen, Cyano, C₁-C₄-Alkyl, C₁-C₄-Alkoxy, C₁-C₄-Alkylthio, C₁-C₄-Halogenalkyl, C₂-C₆-Alkenyl, C₂-C₆-Alkenyloxy und C₁-C₄-Alkoxy-C₁-C₄-alkyl;
eine Phenyl- oder Pyridylgruppe, wobei diese Aromaten gewünschtenfalls noch ein bis drei Substituenten tragen können ausgewählt aus einer Gruppe bestehend aus Halogen, Nitro, Cyano, C₁-C₄-Alkyl, C₁-C₄-Alkoxy, C₁-C₄-Alkylthio, C₁-C₄-Halogenalkyl, C₃-C₆-Alkenyloxy, C₃-C₆-Alkinyloxy und einer Aminogruppe -NR^{a}R^{b}, worin
R^{a} Wasserstoff, C₁-C₄-Alkyl, C₃-C₆-Alkenyl oder C₃-C₆-Alkinyl und
R^{b} Wasserstoff, C₁-C₄-Alkyl, C₃-C₆-Alkenyl, C₃-C₆-Alkinyl, C₁-C₆-Acyl oder Benzoyl, das gewünschtenfalls seinerseits noch ein bis drei Reste tragen kann, ausgewählt aus einer Gruppe bestehend aus Nitro, Cyano, Halogen, C₁-C₄-Alkyl, C₁-C₄-Alkoxy, C₁-C₄-Alkylthio und C₁-C₄-Halogenalkyl;
sowie der landwirtschaftlich brauchbaren Salze und Ester von C₁-C₁₀-Carbonsäuren und anorganischen Säuren der Verbindungen I.

2. Optisch aktive Cyclohexenonoximether I gemäß Anspruch 1 mit R-Konfiguration im Oximetherteil.

3. Verfahren zur Herstellung von Mischungen aus optisch aktiven Cyclohexenonoximethern der Formel I gemäß Anspruch 1, dadurch gekennzeichnet, daß man ein Cyclohexenon der Formel II 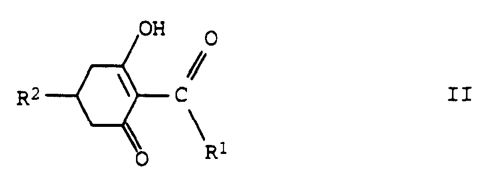 in an sich bekannter Weise in einem inerten organischen Lösungsmittel mit einer Mischung aus optisch aktiven Hydroxylaminen der Formel III 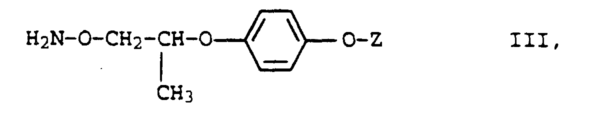 oder mit einem Salz der entsprechenden Hydroxylamine umsetzt.

4. Herbizides Mittel, enthaltend inerte Zusatzstoffe und eine herbizid wirksame Menge einer Mischung aus optisch aktiven Cyclohexenonoximethern der Formel I gemäß Anspruch 1.

5. Verfahren zur Bekämpfung von unerwünschtem Pflanzenwuchs, dadurch gekennzeichnet, daß man eine herbizid wirksame Menge einer Mischung aus optisch aktiven Cyclohexenonoximethern I gemäß Anspruch 1 auf die Pflanzen, deren Lebensraum oder ihr Saatgut einwirken läßt.

6. Mischungen aus optisch aktiven Hydroxylaminen mit R- und S-Konfiguration der Formel III 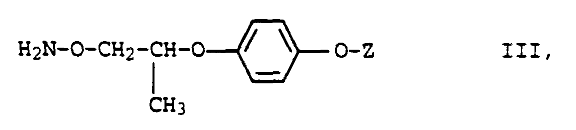 in der Z eine der folgenden Gruppen bedeutet: 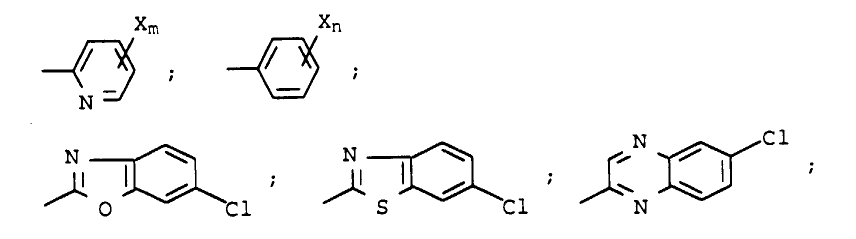 wobei
X für Halogen oder C₁-C₄-Halogenalkyl,
m für 0 bis 3 oder 1 bis 4 für den Fall, daß alle X Halogen bedeuten, und
n für 0 bis 3 oder 1 bis 5 für den Fall, daß alle X Halogen bedeuten,
stehen.

7. Optisch aktive Hydroxylamine III gemäß Anspruch 6 mit R-Konfiguration.

8. Verfahren zur Herstellung von Mischungen aus optisch aktiven Hydroxylaminen der Formel III gemaß Anspruch 6, dadurch gekennzeichnet, daß man eine Mischung aus optisch aktiven 2-Phenoxypropyl-Verbindungen mit R- und S-Konfiguration der Formel IV 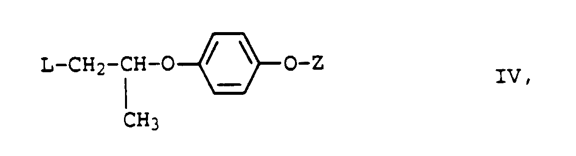 in der L für eine nukleophil substituierbare Abgangsgruppe steht,
in Gegenwart einer Base mit einem cyclischen Hydroxyimid der Formel V 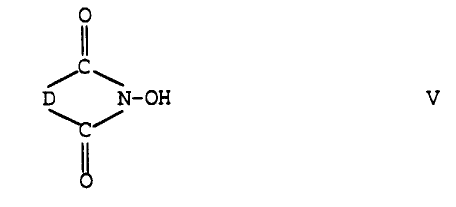 in der D für C₂- oder C₃-Alkylen, C₂-Alkenylen oder einen fünf- oder sechsgliedrigen Ring steht, der gesättigt oder ein- bis dreifach ungesättigt sein kann und der gewünschtenfalls ein Stickstoffatom als Ringglied enthalten kann,
umsetzt und das Verfahrensprodukt VI 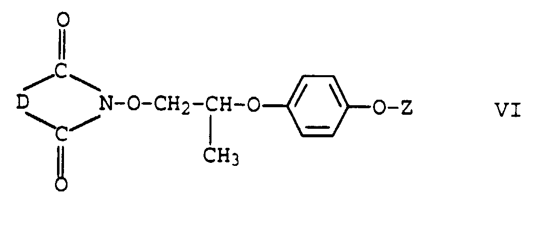 sauer oder basisch spaltet.

9. Verfahren nach Anspruch 8, dadurch gekennzeichnet, daß man das Hydroxylamin III aus dem Verfahrensprodukt VI mittels Ethanolamin freisetzt.

## Claims

1. A mixture of optically active cyclohexenone oxime ethers having the R and S configurations in the oxime ether moiety, of the formula I 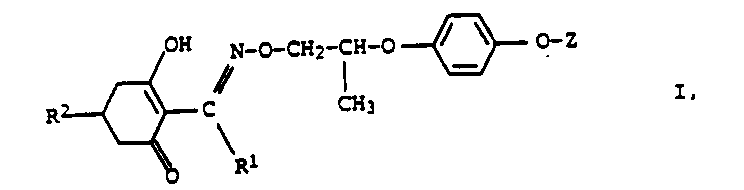 where
R¹ is C₁-C₆ alkyl;
Z is one of the following groups: 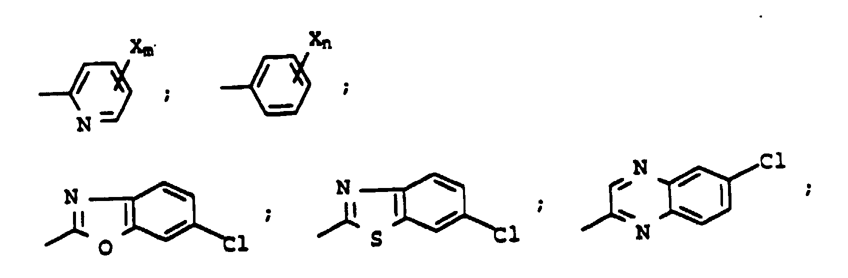
X is halogen or C₁-C₄-haloalkyl;
m is from 0 to 3 or is from 1 to 4 if all X are halogen;
n is from 0 to 3 or is from 1 to 5 if all X are halogen;
R² is C₁-C₄-alkoxy-C₁-C₆-alkyl or C₁-C₄-alkylthio-C₁-C₆-alkyl;
C₃-C₇-cycloalkyl or C₅-C₇-cycloalkenyl, where these groups may, if desired, carry from one to three substitutents selected from the group consisting of C₁-C₄alkyl, C₁-C₄-alkoxy, C₁-C₄-alkylthio, C₁-C₄-haloalkyl, hydroxyl and halogen;
a 5-membered saturated heterocyclic radical which contains one or two oxygen atoms and/or sulfur atoms as hetero atoms and which, if desired, may furthermore carry from one to three substituents selected from the group consisting of C₁-C₄-alkyl, C₁-C₄-alkoxy, C₁-C₄-alkylthio and C₁-C₄-haloalkyl;
a 6- or 7-membered saturated or mono- or diunsaturated heterocyclic radical which contains one or two oxygen or sulfur atoms or one oxygen and one sulfur atom as hetero atoms, where the heterocyclic radical may, if desired, furthermore carry from one to three substituents selected from the group consisting of hydroxyl, halogen, C₁-C₄-alkyl, C₁-C₄-alkoxy, C₁-C₄-alkylthio and C₁-C₄-haloalkyl;
a 5-membered heteroaromatic radical containing from one to three hetero atoms selected from the group consisting of one or two nitrogen atoms and one oxygen or sulfur atom, where the heteroaromatic radical may, if desired, furthermore carry from one to three substituents selected from the group consisting of halogen, cyano, C₁-C₄-alkyl, C₁-C₄-alkoxy, C₁-C₄-alkylthio and C₁-C₄-haloalkyl, C₂-C₆-alkenyl, C₂-C₆-alkenyloxy and C₁-C₄-alkoxy-C₁-C₄-alkyl;
phenyl or pyridyl, where these aromatic radicals may, if desired, furthermore carry from one to three substituents selected from the group consisting of halogen, nitro, cyano, C₁-C₄-alkyl, C₁-C₄-alkoxy, C₁-C₄-alkylthio and C₁-C₄-haloalkyl, C₃-C₆-alkenylkoxy C₃-C₆-alkynyloxy and an amino group -NR^{a}R^{b}, where
R^{a} is hydrogen, C₁-C₄-alkyl, C₃-C₆-alkenyl or C₃-C₆-alkynyl and
R^{b} is hydrogen, C₁-C₄-alkyl, C₃-C₆-alkenyl, C₃-C₆-alkynyl, C₁-C₆-acyl or benzoyl which, if desired, may in turn furthermore carry from one to three radicals selected from the group consisting of nitro, cyano, halogen, C₁-C₄-alkyl, C₁-C₄-alkoxy, C₁-C₄-alkylthio and C₁-C₄-haloalkyl;
or the agriculturally useful salts or esters of the compounds I with C₁-C₁₀-carboxylic acids or inorganic acids.

2. An optically active cyclohexenone oxime ether I as claimed in claim 1, having the R configuration in the oxime ether moiety.

3. A process for the preparation of a mixture of optically active cyclohexenone oxime ethers of the formula I as claimed in claim 1, wherein a cyclohexenone of the formula II 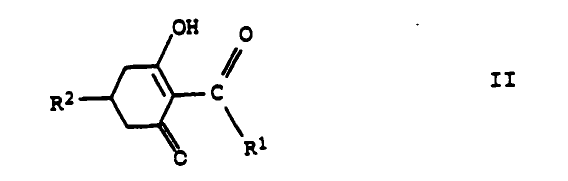 is reacted in a manner known per se, in an inert organic solvent, with a mixture of optically active hydroxylamines of the formula III 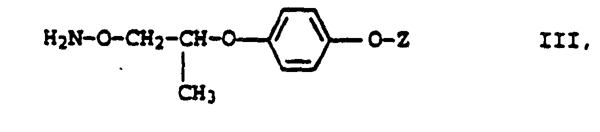 or with a salt of the corresponding hydroxylamines.

4. A herbicide containing inert additives and a herbicidal amount of a mixture of optically active cyclohexenone oxime ethers of the formula I as claimed in claim 1.

5. A method for controlling undesirable plant growth, wherein a herbicidal amount of a mixture of optically active cyclohexenone oxime ethers I as claimed in claim 1 is allowed to act on the plants, their habitat or their seeds.

6. A mixture of optically active hydroxylamines having the R and S configurations, of the formula III 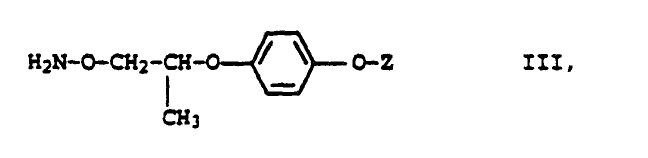 where Z is one of the following groups: 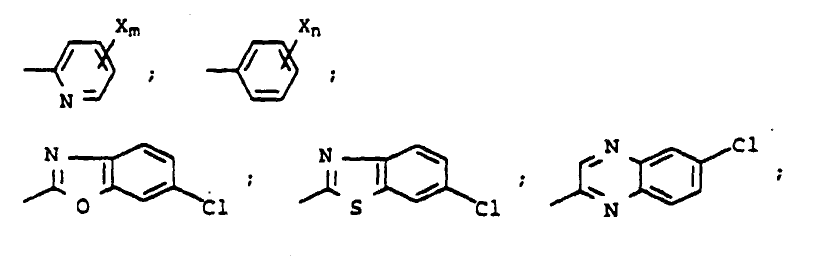 in which
X is halogen or C₁-C₄-haloalkyl,
m is from 0 to 3 or is from 1 to 4 if all X are halogen and
n is from 0 to 3 or is from 1 to 5 if all X are halogen.

7. An optically active hydroxylamine III as claimed in claim 6, having the R configuration.

8. A process for the preparation of a mixture of optically active hydroxylamines of the formula III as claimed in claim 6, wherein a mixture of optically active 2-phenoxypropyl compounds having the R and S configurations of the formula IV 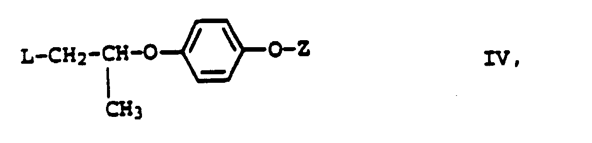 where L is a nucleophilically substitutable leaving group, is reacted, in the presence of a base, with a cyclic hydroxyimide of the formula V 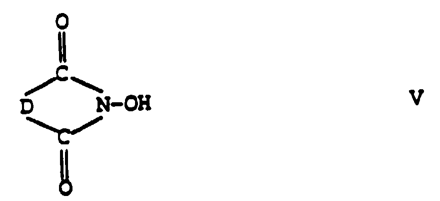 where D is C₂- or C₃-alkylene, C₂-alkenylene or a five- or six-membered ring which may be saturated or mono- to triunsaturated and which, if desired, may contain a nitrogen atom as a ring member,
and the product VI 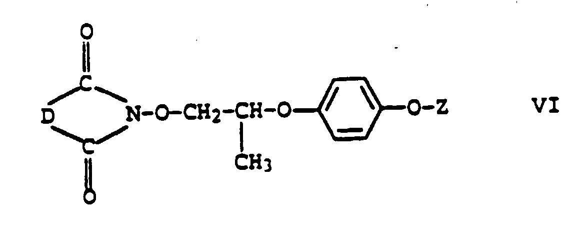 is cleaved under acidic or basic conditions.

9. A process as claimed in claim 8, wherein the hydroxylamine III is liberated from the product VI by means of ethanolamine.

## Revendications

1. Mélanges d'éthers de cyclohexénonoximes possédant l'activité optique, en configurations R et S dans la partie éther d'oxime, répondant à la formule générale I 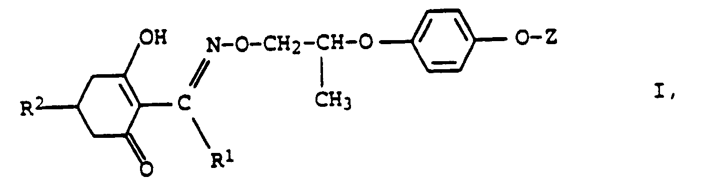 dans laquelle les symboles ont les significations suivantes :
R¹ représente un groupe alkyle en C1-C6 ;
Z représente l'un des groupes suivants : 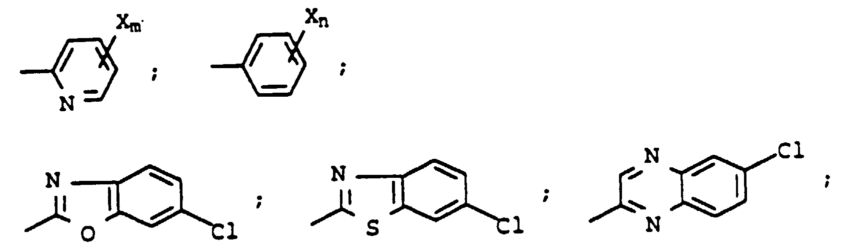
X représente un halogène, un groupe halogénoalkyle en C1-C4 ;
m est un nombre allant de 0 à 3 ou de 1 à 4 lorsque tous les symboles X représentent des halogènes ;
n est un nombre allant de 0 à 3 ou de 1 à 5 lorsque tous les symboles X représentent des halogènes ;
R² représente
un groupe (alcoxy en C1-C4)alkyle en C1-C6 ou (alkylthio en C1-C4)alkyle en C1-C6 ;
un groupe cycloalkyle en C3-C7 ou cycloalcényle en C5-C7, ces groupes pouvant porter le cas échéant un à trois substituants choisis parmi les groupes alkyle en C1-C4, alcoxy en C1-C4, alkylthio en C1-C4, halogénoalkyle en C1-C4, hydroxy et les halogènes ;
un hétérocycle saturé à cinq chaînons contenant, en tant qu'hétéroatomes, un ou deux atomes d'oxygène et/ou de soufre et qui peut encore porter le cas échéant un à trois substituants choisis parmi les groupes alkyle en C1-C4, alcoxy en C1-C4, alkylthio en C1-C4 et halogénoalkyle en C1-C4 ;
un hétérocycle saturé ou mono- ou di-insaturé à six chaînons qui contient un ou deux atomes d'oxygène ou de soufre ou un atome d'oxygène et un atome de soufre, l'hétérocycle pouvant encore porter le cas échéant un à trois substituants choisis parmi les groupes hydroxy, les halogènes, les groupes alkyle en C1-C4, alcoxy en C1-C4, alkylthio en C1-C4 et halogénoalcoxy en C1-C4 ;
un radical hétéroaromatique à cinq chaînons contenant un à trois hétéroatomes consistant en un ou deux atomes d'azote et un atome d'oxygène ou de soufre,
le radical hétéroaromatique pouvant encore porter le cas échéant un à trois substituants choisis parmi les halogènes, les groupes cyano, alkyle en C1-C4, alcoxy en C1-C4, alkylthio en C1-C4, halogénoalcoxy en C1-C4, alcényle en C2-C6, alcényloxy en C2-C6, et (alcoxy en C1-C4)-alkyle en C1-C4 ;
un groupe phényle ou pyridyle, ces groupes aromatiques pouvant porter le cas échéant un à trois substituants choisis parmi les halogènes, les groupes nitro, cyano, alkyle en C1-C4, alcoxy en C1-C4, alkylthio en C1-C4, halogénoalkyle en C1-C4, alcényloxy en C3-C6, alcynyloxy en C3-C6 et amino -NR^{a}R^{b}, dans lequel
R^{a} représente l'hydrogène, un groupe alkyle en C1-C4, alcényle en C3-C6 ou alcynyle en C3-C6 et
R^{b} représente l'hydrogène, un groupe alkyle en C1-C4, alcényle en C3-C6, alcynyle en C3-C6, acyle en C1-C6 ou benzoyle, qui peut lui-même porter encore un à trois substituants choisis parmi les groupes nitro, cyano, les halogènes, les groupes alkyle en C1-C4, alcoxy en C1-C4, alkylthio en C1-C4 et halogénoalkyle en C1-C4 ;
et les sels et esters d'acides carboxyliques en C1-C10 et d'acides minéraux des composés I convenant pour des applications agricoles.

2. Ethers de cyclohexénonoximes I selon la revendication 1, possédant l'activité optique, en configuration R dans la partie éther d'oxime.

3. Procédé de préparation des mélanges d'éthers de cyclohexénonoximes possédant l'activité optique, de formule I de la revendication 1, caractérisé par le fait que l'on fait réagir une cyclohexénone de formule II 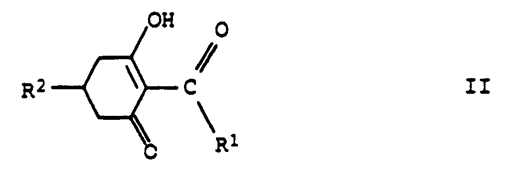 de manière connue en soi, dans un solvant organique inerte, avec un mélange d'hydroxylamines possédant l'activité optique et répondant à la formule III 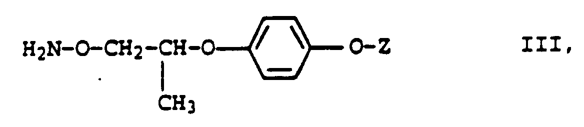 ou avec un sel des hydroxylamines correspondantes.

4. Produit herbicide contenant des additifs inertes et une quantité herbicide efficace d'un mélange d'éthers de cyclohexénonoximes, possédant l'activité optique, de formule I de la revendication 1.

5. Procédé pour combattre les croissances de végétaux indésirables, caractérisé par le fait que l'on fait réagir une quantité herbicide efficace d'un mélange d'éthers de cyclohexénonoximes possédant l'activité optique I de la revendication 1 sur les végétaux, leur habitat ou leurs semences.

6. Mélanges d'hydroxylamines possédant l'activité optique, en configuration R et S, de formule III 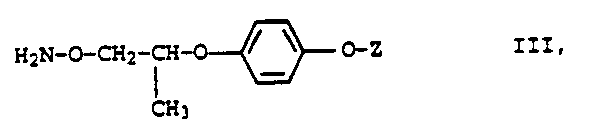 dans laquelle Z représente l'un des groupes suivants : 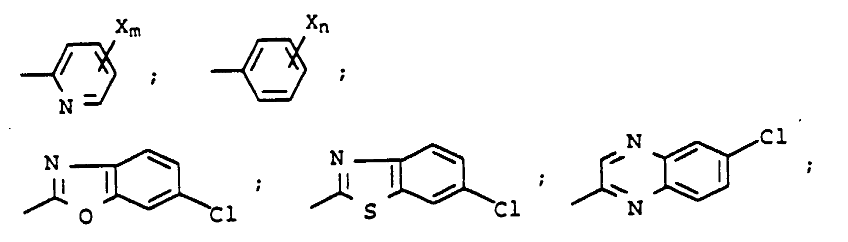 dans lesquels
X représente un halogène ou un groupe halogénoalkyle en C1-C4,
m est un nombre allant de 0 à 3 ou de 1 à 4 lorsque tous les symboles X représentent des halogènes et
n est un nombre allant de 0 à 3 ou de 1 à 5 lorsque tous les symboles X représentent des halogènes.

7. Hydroxylamines possédant l'activité optique, de formule III de la revendication 6, en configuration R.

8. Procédé de préparation des mélanges d'hydroxylamines possédant l'activité optique de formule III de la revendication 6, caractérisé par le fait que l'on fait réagir un mélange de dérivés 2-phénoxypropyliques possédant l'activité optique, en configuration R et S, de formule IV 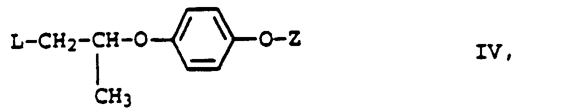 dans laquelle L représente un groupe éliminable apte à une substitution nucléophile, en présence d'une base,
avec un hydroxyimide cyclique de formule V 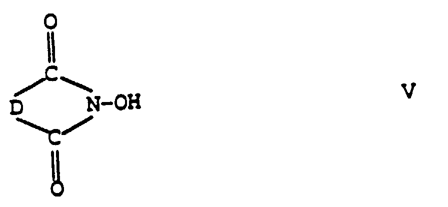 dans laquelle D représente un groupe alkylène en C2 ou C3, alcényle en C2 ou un cycle à cinq ou six chaînons, saturé ou mono- à tri-insaturé et qui peut contenir le cas échéant un atome d'azote en tant que chaînon cyclique,
ce qui donne le composé VI 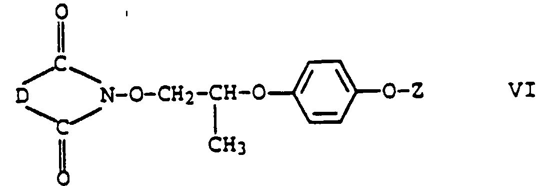 qu'on soumet à scission acide ou basique.

9. Procédé selon la revendication 8, caractérisé par le fait qu'on libère l'hydroxylamine III à partir du composé VI à l'aide de l'éthanolamine.
